# EUROPÄISCHE PATENTSCHRIFT

(11) **EP 2 691 523 B1**
(45) Veröffentlichungstag und Bekanntmachung des Hinweises auf die Patenterteilung: **19.07.2017**
(21) Anmeldenummer: 12711166.4
(22) Anmeldetag: 29.03.2012
(51) Int. Cl.: C12N 15/11, C12N 15/115, G01N 33/53, G01N 33/558, G01N 33/94

(54) **AMINOGLYKOSID-SPEZIFISCHE APTAMERE**
AMINOGLYCOSIDE-SPECIFIC APTAMERS
APTAMÈRES SPÉCIFIQUES D'AMINOGLYCOSIDES

(30) Priorität: 31.03.2011 DE 102011006612
(43) Veröffentlichungstag der Anmeldung: 05.02.2014
(73) Patentinhaber: Helmholtz-Zentrum für Umweltforschung GmbH - UFZ, 04318 Leipzig (DE)
(72) Erfinder: NIKOLAUS, Nadia, 04179 Leipzig (DE); PRANGE, Anja, 04277 Leipzig (DE); STOLTENBURG, Regina, 04157 Leipzig (DE); STREHLITZ, Beate, 04279 Leipzig (DE)
(74) Vertreter: Beyer, Andreas
(86) Internationale Anmeldenummer: PCT/EP2012/055651
(87) Internationale Veröffentlichungsnummer: WO 2012/130948

(56) Entgegenhaltungen:
- US-A1- 2004 171 095
- ROWE A.A. ET AL.: "Reagentless measurement of Aminoglycoside Antibiotics in blood serum via an electrochemical, Ribonucleic Acid Aptamer-based biosensor", ANALYTICAL CHEMISTRY, Bd. 82, Nr. 17, 1. September 2010 (2010-09-01), Seiten 7090-7095, XP55028652, ISSN: 0003-2700, DOI: 10.1021/ac101491d & ROWE A.A. et al.: "Additional Information", , 1. September 2010 (2010-09-01), XP002677044, Gefunden im Internet: URL:http://www.ncbi.nlm.nih.gov/pmc/articl es/PMC3082472/?tool=pubmed#SD1 [gefunden am 2012-05-31]
- WANG Y. AND RANDO R.R.: "Specific binding of aminoglycoside antibiotics to RNA", CHEMISTRY AND BIOLOGY, CURRENT BIOLOGY, LONDON, GB, Bd. 2, Nr. 5, 1. Mai 1995 (1995-05-01), Seiten 281-290, XP002926258, ISSN: 1074-5521, DOI: 10.1016/1074-5521(95)90047-0
- WANG YONG ET AL.: "RNA molecules that specifically and stoichiometrically bind aminoglycoside antibiotics with high affinities", BIOCHEMISTRY, AMERICAN CHEMICAL SOCIETY, US, Bd. 35, Nr. 38, 1. Januar 1996 (1996-01-01), Seiten 12338-12346, XP002314739, ISSN: 0006-2960, DOI: 10.1021/BI960878W
- JIANG L. AND PATEL D.J.: "Solution structure of the tobramycin-RNA aptamer complex.", NATURE STRUCTURAL BIOLOGY SEP 1998 LNKD- PUBMED:9731769, Bd. 5, Nr. 9, September 1998 (1998-09), Seiten 769-774, XP002677045, ISSN: 1072-8368
- KEITA HAMASAKI ET AL.: "Minimal RNA constructs that specifically bind Aminoglycoside Antibiotics with high affinities", BIOCHEMISTRY, Bd. 37, Nr. 2, 1. Januar 1998 (1998-01-01) , Seiten 656-663, XP55028656, ISSN: 0006-2960, DOI: 10.1021/bi971095t
- JUNHYEONG CHO ET AL.: "The binding site of a specific Aminoglycoside binding RNA molecule", BIOCHEMISTRY, Bd. 37, Nr. 14, 1. April 1998 (1998-04-01) , Seiten 4985-4992, XP55028658, ISSN: 0006-2960, DOI: 10.1021/bi972757h
- MORSE D.P.: "Direct selection of RNA beacon aptamers", BIOCHEMICAL AND BIOPHYSICAL RESEARCH COMMUNICATIONS, ACADEMIC PRESS INC. ORLANDO, FL, US, Bd. 359, Nr. 1, 2. Juni 2007 (2007-06-02), Seiten 94-101, XP022103148, ISSN: 0006-291X, DOI: 10.1016/J.BBRC.2007.05.072 & MORSE D.P.: "Supplementary data", , 20. Juli 2007 (2007-07-20), XP002677046, Gefunden im Internet: URL:http://www.ncbi.nlm.nih.gov/pmc/articl es/PMC2030492/?tool=pubmed [gefunden am 2012-06-01]
- VANDENENGEL J.E. AND MORSE D.P.: "Mutational analysis of a signaling aptamer suggests a mechanism for ligand-triggered structure-switching", BIOCHEMICAL AND BIOPHYSICAL RESEARCH COMMUNICATIONS, Bd. 378, Nr. 1, Januar 2009 (2009-01), Seiten 51-56, XP25741815, ISSN: 0006-291X & VANDENENGEL J.E. and MORSE D.P.: "Supplementary Data", , 2. Januar 2009 (2009-01-02), XP002677047, Gefunden im Internet: URL:http://www.sciencedirect.com/science/a rticle/pii/S0006291X08021608 [gefunden am 2012-05-31]
- KWON M. ET AL.: "In vitro selection of RNA against Kanamycin B", MOLECULES AND CELLS, SEOUL, KR, Bd. 11, Nr. 3, 30. Juni 2001 (2001-06-30), Seiten 303-311, XP009034668, ISSN: 1016-8478
- HERMANN .T AND WESTHOF E.: "Docking of cationic antibiotics to negatively charged pockets in RNA folds", JOURNAL OF MEDICINAL CHEMISTRY, AMERICAN CHEMICAL SOCIETY, US, Bd. 42, Nr. 7, 8. April 1999 (1999-04-08), Seiten 1250-1261, XP002229177, ISSN: 0022-2623, DOI: 10.1021/JM981108G
- PATEL D.J. ET AL.: "Structure, recognition and adaptive binding in RNA aptamer complexes", JOURNAL OF MOLECULAR BIOLOGY, ACADEMIC PRESS, UNITED KINGDOM, Bd. 272, Nr. 5, 10. Oktober 1997 (1997-10-10), Seiten 645-664, XP004453689, ISSN: 0022-2836, DOI: 10.1006/JMBI.1997.1281
- LICONG JIANG ET AL.: "Saccharide-RNA recognition in an aminoglycoside antibiotic-RNA aptamer complex", CHEMISTRY AND BIOLOGY, CURRENT BIOLOGY, LONDON, GB, Bd. 4, Nr. 1, 1. Januar 1997 (1997-01-01), Seiten 35-50, XP009156477, ISSN: 1074-5521
- COWAN J.A. ET AL: "Recognition of a cognate RNA aptamer by neomycin B: Quantitative evaluation of hydrogen bonding and electrostatic interactions", NUCLEIC ACIDS RESEARCH, Bd. 28, Nr. 15, 1. August 2000 (2000-08-01), Seiten 2935-2942, XP002677048, ISSN: 0305-1048
- GOERTZ P.W. ET AL.: "Automated selection of aminoglycoside aptamers", JALA - JOURNAL OF THE ASSOCIATION FOR LABORATORY AUTOMATION 200406 US LNKD- DOI:10.1016/J.JALA.2004.04.008, Bd. 9, Nr. 3, Juni 2004 (2004-06), Seiten 150-154, XP002677049, ISSN: 1535-5535
- LATO S.M. AND ELLINGTON A.D.: "Screening chemical libraries for nucleic-acid-binding drugs by in vitro selection: A test case with lividomycin", MOLECULAR DIVERSITY, Bd. 2, Nr. 1-2, 1996, Seiten 103-110, XP008152377, ISSN: 1381-1991

## Beschreibung

Die vorliegende Erfindung betrifft ein Aptamer, das an eine oder mehrere Verbindungen aus der Gruppe der Aminoglykoside, bindet, Verwendungen des Aptamers sowie Verfahren zum Nachweis und Anreicherung von Verbindungen aus der Gruppe der Aminoglykoside, in denen das Aptamer eingesetzt wird.

Aminoglykoside sind Oligosaccharide, die einen Aminozucker enthalten. Bei einem Aminozucker ist eine oder mehrere Hydroxygruppen durch eine Aminogruppe ersetzt, wobei ein oder mehrere Wasserstoffatome der Aminogruppe durch einen Substituenten substituiert sein können. Die wichtigste Anwendung von Aminoglykosiden ist die Anwendung als Antibiotika.

Kanamycine sind strukturell eng verwandte Aminoglykoside. Kanamycin A gehört zur Gruppe der Kanamycine. Handelsübliches Kanamycin enthält hauptsächlich Kanamycin A, ist aber ein Gemisch aus den Kanamycinen A, B und C. Es ist ein verschreibungspflichtiges Aminoglykosid-Antibiotikum aus Streptomyceten (Streptomyces kanamyceticus). Des Weiteren gehören Tobramycin und Dibekacin zur Gruppe der Kanamycine. Weitere Aminoglykoside, die auch als Antibiotika eingesetzt werden, sind Streptomycin, Neomycin, Paromomycin, Gentamicin, Spectinomycin sowie die halbsynthetischen Aminogylcoside Amikacin und Netilmicin.

In Deutschland wird Kanamycin in der Humanmedizin als Sulfatsalz in Form von Augentropfen und -salben zur lokalen Behandlung bakterieller Infektionen des Auges (z. B. bei einer Bindehautentzündung) eingesetzt. In den USA sind von Kanamycin auch Darreichungsformen zur oralen und parenteralen Anwendung im Handel.

In der Veterinärmedizin wird Kanamycin als Reserveantibiotikum zur Behandlung von Magen-Darm-Infektionen durch Kanamycin-empfindliche Erreger bei Hunden und Katzen sowie in Kombination mit Spiramycin bei akuten und chronischen, gegen andere Therapien resistente Mastitiden verwendet. In der Humanmedizin dient Kanamycin als Reserveantibiotikum unter anderem für die Behandlung multiresistenter Tuberkulose.

Weit verbreitet sind Kanamycine auch in der Molekularbiologie als Selektionsantibiotikum im Rahmen von Klonierungsexperimenten. Bei diesen gentechnischen Arbeiten werden Mikroorganismen, vornehmlich Escherichia coli, mit Plasmiden transformiert, die zusätzlich zu den interessierenden Genen mit Resistenzgenen, beispielsweise gegen Kanamycin ausgestattet sind. Somit wird in Klonierungsexperimenten eine Auslese hinsichtlich positiver Transformanten (plasmidtragende E.coli) erlaubt, indem man diese in kanamycinhaltigen Medien kultiviert.

Im Gegensatz zu den meisten anderen Antibiotika wirkt Kanamycin auch auf Pflanzen toxisch. Entsprechend kann beim Arbeiten mit transgenen Pflanzen und einer entsprechenden Versuchsdurchführung auch auf Kanamycin-Selektionsmedien zurückgegriffen werden. Aufgrund dieser vielfältigen Anwendungen gelangt Kanamycin über das Abwasser in die Umwelt. Wird es in Kläranlagen nicht vollständig abgebaut, oder gelangt es aus der Tierhaltung über das Oberflächen- und Grundwasser in das Trinkwasser, kann es zu einer dauerhaften Belastung des Menschen mit dem Antibiotikum kommen, was wiederum die Ursache für Antibiotikaresistenzen sein kann.

Die Bestimmung oder Messung von Kanamycin und insbesondere Kanamycin A im Trinkwasser, in Oberflächen- und Grundwasser und in Kläranlagen ist daher sehr wichtig. Ein einfaches Testsystem für Kanamycin A würde die Möglichkeit bieten, Antibiotika aus der Gruppe der Kanamycine einfach und schnell vor Ort zu messen.

Die Messung der Antibiotika erfolgt bisher mit aufwändigen Methoden wie GC/MS oder HPLC im Labor, wofür teure Messgeräte und gut ausgebildetes Personal erforderlich sind, wodurch die Messungen teuer sind und den Transport der Proben in das Labor erfordern.

A.A. Rowe et al. "Reagentless Measurement of Aminoglycoside Antibiotics in Blood Serum via an Electrochemical, Ribonucleic Acid Aptamer-Based Biosensor", https://www.ncbi.nlm.nih.gov/pmc/articles/PMC3082472/?tool=pubmed#SD1 Anal Chem. 2010 Sep 1; 82(17): 7090-7095 (auch unter https://www.ncbi.nlm.nih.gov/pmc/articles/PMC3082472/?tool=pubmed#SD1) beschreiben einen DNA-Aptamersensor zur Detektion von Aminoglykosidantibiotika. Nachteilig bei diesem Sensor ist, dass er in Blutserum stabil ist, aber seine geringe Affinität sein Detektionslimit über den therapeutisch relevanten Bereich hebt. Ein entsprechender RNA-Aptamersensor ist wiederum instabil.

Aufgabe der vorliegenden Erfindung war es, spezifische Substanzen zur Verfügung zu stellen, die einen schnellen, einfachen und zuverlässigen Nachweis von Kanamycin A und anderer Verbindungen aus der Gruppe der Kanamycine ermöglichen.

Die Aufgabe wird mit einem Aptamer nach Anspruch 1, gelöst, das das an eine oder mehrere Verbindungen aus der Gruppe der Aminoglykoside bindet, insbesondere an eine oder mehrere Verbindungen aus der Gruppe der Kanamycine oder Paromomycin. Insbesondere ist das erfindungsgemäße Aptamer spezifisch für Verbindungen aus der Gruppe der Aminoglykoside, insbesondere für eine Verbindung aus der Gruppe der Kanamycine oder Paromomycin. Das erfindungsgemäße Aptamer ist ein Nukleinsäure-Aptamer, insbesondere ein einzelsträngiges DNA(ssDNA)- oder ein RNA-Aptamer.

Der Begriff "Aminoglykoside" bezeichnet Oligosaccharide, die einen Aminozucker enthalten. Insbesondere umfasst sind Aminoglykoside, die als Antibiotika eingesetzt werden.

Bevorzugte Aminoglykoside, auf die sich die nachfolgende Offenbarung insbesondere bezieht, und die ein besonders bevorzugtes Target für die erfindungsgemäßen Aptamere darstellen, sind die Gruppe der Kanamycine, Streptomycin, Neomycin, Paromomycin, Gentamicin, Spectinomycin, Amikacin, Netilmicin, Apramycin und Geneticin. Besonders bevorzugte Aminoglykoside sind die Gruppe der Kanamycine und Paromomycin. Die Gruppe der Kanamycine umfasst Kanamycin A, Kanamycin B, Kanamycin C, Tobramycin und Dibekacin.

Der allgemeine Begriff "Aptamere" bezeichnet im Stand der Technik kurze einzelsträngige Nukleinsäure-Oligomere, auch bezeichnet als Oligonukleotide, die spezifisch an eine Zielstruktur oder ein Zielmolekül, auch bezeichnet als Target, binden können, beispielsweise an ein Protein, an niedermolekulare Verbindungen, wie organische Substanzen, Aminosäuren und Antibiotika, Nukleinsäuren, Viruspartikel oder (Mikro)Organismen. Die Aptamer-Target-Bindung erfolgt beispielsweise über die Strukturkompatibilität, so genannte "Stacking interactions" bei aromatischen Ringstrukturen (Stapelkräfte durch Elektronenwechselwirkung mit Nachbarbasen), elektrostatische Wechselwirkungen (z.B. Van der Waals-, Ionen-, Dipolkräfte) und Wasserstoffbrückenbindungen.

Auch Aptamere mit Peptidstruktur sind bekannt, wobei sich die vorliegende Erfindung ausschließlich auf Nukleinsäure-Aptamere bezieht. Somit bezeichnet der Begriff "Aptamere" nachfolgend Nukleinsäure-Aptamere. Bei Nukleinsäure-Aptameren unterscheidet man beispielsweise DNA-Aptamere, gebildet aus einzelsträngiger DNA (ssDNA), und RNA-Aptamere. Aptamere zeichnen sich durch die Ausbildung einer spezifischen dreidimensionalen Struktur, die von der Nukleinsäuresequenz abhängt, aus. Diese Struktur befähigt Aptamere, analog einer Antigen-Antikörper-Bindung Zielstrukturen passgenau zu binden. Eine bestimmte Nukleinsäuresequenz eines Aptamers kann bei definierten Bedingungen eine dreidimensionale Struktur aufweisen, die spezifisch für eine definierte Zielstruktur (Target) ist. Die dreidimensionale Struktur eines Aptamers entsteht u.a. infolge von intramolekularen Basenpaarungen nach Watson und Crick und über Hoogsteen-Basenpaarungen (Quadruplex).

Erfindungsgemäße Aptamere können beispielsweise mit dem SELEX-Verfahren (Systematic Evolution of Ligands by Exponential Enrichment) erhalten werden. Grundlegende Arbeiten zum SELEX-Verfahren stammen von Tuerk and Gold, Science 249 (1990) 505-510, sowie Ellington and Szostak, Nature 346 (1990) 818-822. SELEX Verfahren sind weiterhin offenbart in US 5,567,588 und US 5,270,163.

Im SELEX Verfahren wird zunächst eine kombinatorische Zufallsbibliothek, bestehend aus einzelsträngigen DNA (ssDNA)-Oligonukleotiden erzeugt. Die Oligonukleotide weisen einen internen variablen Bereich auf, mit beispielsweise 40-60 Nukleotiden, der am 5' und 3' Ende von Primerregionen flankiert wird. Die Primerregionen dienen als Primerbindungsstellen für eine PCR Amplifikation. Kombinatorische Zufallsbibliotheken können von kommerziellen Anbietern bezogen werden. Die Variabilität einer Bibliothek liegt beispielsweise im Bereich von ca. 10¹⁵ verschiedenen Molekülen. Ausgehend von der einzelsträngigen DNA-Oligonukleotid-Bibliothek werden über verschiedene Selektions- und Amplifikationsschritte zyklusweise die Oligonukleotide angereichert, die am besten an das Target binden. Jeder Zyklus besteht aus folgenden Teilschritten:
a) Bindung der Oligonukleotide an das Target,
b) Waschen der Oligonukleotid-Target-Komplexe zur Entfernung von ungebundenen Oligonukleotiden,
c) Elution der am Target gebundenen Oligonukleotide,
d) Amplifikation der eluierten Oligonukleotide mittels PCR,
e) Reinigung der relevanten ssDNA-Oligonukleotide aus dem PCR-Produkt

Nach jedem Zyklus wird der selektierte und angereicherte Oligonukleotidpool als Ausgangsmaterial für einen nächsten Zyklus genutzt. Vorteilhafterweise werden 8 bis 12 Zyklen durchlaufen.

Ein SELEX Verfahren zur Isolierung erfindungsgemäßer Aptamere ist in den beigefügten Beispielen genauer beschrieben. Ein beispielhaftes Verfahren zur Auffindung von erfindungsgemäßen Aptameren ist das sogenannte Capture-SELEX Verfahren, das in den beigefügten Beispielen beschrieben ist.

Nach Analyse der Sequenz können erfindungsgemäße Aptamere sowie Varianten, Mutanten, Fragmente und Derivate davon, welche nachfolgend noch beschrieben werden, mit üblichen Techniken der chemischen DNA- und RNA-Synthese hergestellt werden, die dem Fachmann bekannt sind. Ist die Sequenz eines DNA Aptamers bekannt, so kann ein RNA Aptamer mit der gleichen Sequenz hergestellt werden. Ferner können die Bindungseigenschaften einzelner Aptamere an das Target untersucht werden. Vorzugsweise sind die erfindungsgemäßen Aptamere synthetische Oligonukleotide, die vorzugsweise nach dem soeben beschriebenen SELEX Verfahren aus einer zugrunde gelegten synthetischen, kombinatorischen Oligonukleotid-Bibliothek selektiert wurden. Die Erfindung betrifft ein Aptamer, das an eine oder mehrere Verbindungen aus der Gruppe der Aminoglykoside, insbesondere an eine Verbindung aus der Gruppe der Kanamycine oder Paromomycin, bindet und das ausgewählt ist aus der Gruppe bestehend aus
a) einem Aptamer umfassend, oder bestehend aus, eine(r) Nukleinsäuresequenz, die ausgewählt ist aus der Gruppe bestehend aus SEQ ID NO: 45, 1-10, 50, 23-26, 52, 29-31, 48, 17-19, 51, 27, 28, 53, 32, 33, 54, 34, 35, und 55-58, mit der Maßgabe, dass Thymin durch Uracil ersetzt sein kann,
b) einem Aptamer, dessen Nukleinsäuresequenz eine Identität von mindestens 70% mit der Nukleinsäuresequenz eines Aptamers aus a) aufweist
c) einem Aptamer, das mit dem komplementären Strang eines Aptamers aus a) unter stringenten Bedingungen hybridisiert,
d) einem Aptamer bei dem gegenüber einem Aptamer aus a) ein oder mehrere Nukleotide substituiert, deletiert, insertiert und/oder angefügt sind, wobei dieses Aptamer eine Identität von mindestens 70% mit der Nukleinsäuresequenz eines Aptamers aus a) aufweist,
e) einem Fragment eines Aptamers nach a), b), c) oder d), und
f) einem Derivat eines Aptamers nach a), b), c), d) oder e), das eine chemische Struktur aufweist, die in natürlicher DNA oder RNA nicht vorkommt.

Die Funktionalität der Aptamere aus a) - f), also die Bindung an Verbindungen aus der Gruppe der Aminoglykoside kann mit einer Sekundärstrukturanalyse der Aptamere, abgeschätzt werden. Auch die Funktionalität sonstiger in dieser Beschreibung genannter Varianten kann so abgeschätzt werden. Die Modellierung der möglichen Sekundärstruktur der Aptamere kann unter Nutzung des im Internet frei verfügbaren Programms "mfold" (Version 3.1 oder 3.5) erfolgen. Dieses Programm führt eine Analyse der zweidimensionalen Struktur mit Hilfe einer Energieminimierungsmethode aus (Zuker M., 2003, Nucleic Acid Research 31, 3406-3415). Bei der Faltung der Aptamere kann es zu Stems (doppelsträngige Bereiche), Loops, d.h. Ausschleifungen von einzelsträngigen Bereichen, wie Haarnadel- oder interne Schleifen, und Bulbs, d.h. kleine einzelsträngige Ausbuchtungen in doppelsträngigen Bereichen, kommen. Mit dem Programm mfold wird für eine Aptamer-Nukleotidsequenz die hypothetische Sekundärstruktur bei Standardbedingungen bestimmt (Bindungs-/Faltungstemperatur: 21°C, Ionenstärke des Bindungspuffers: [Na⁺] 100 mM / [Mg²⁺] 2 mM). Bindungspufferzusammensetzung: 100 mM NaCl, 20 mM Tris-HCl, pH 7.6, 2 mM MgCl₂, 5 mM KCl, 1 mM CaCl₂. Der Fachmann kann sehr leicht eine große Anzahl Varianten der konkret mit Sequenzen beschriebenen erfindungsgemäßen Aptamere entwerfen, beispielsweise durch Substitution, Insertion oder Deletion einzelner Basen. Solche Varianten können per Computer, wie oben beschrieben, in großer Zahl und ohne experimentellen Aufwand auf ihre Sekundärstruktur analysiert werden. Wenn eine Übereinstimmung der Sekundärstruktur einer Variante mit der Sekundärstruktur eines konkret mit Sequenz beschriebenen Aptamers vorliegt, ist eine Funktionalität der Variante wahrscheinlich oder bisweilen sehr wahrscheinlich.

Varianten der Aptamere nach Anspruch 1a) werden nachfolgend erläutert.

Von der Erfindung umfasst sind Aptamere, deren Sequenz eine Identität von mindestens 70%, vorzugsweise mindestens 80%, oder mindestens 85%, oder mindestens 90%, oder mindestens 93%, oder mindestens 95%, oder mindestens 96%, oder mindestens 97%, und am meisten bevorzugt mindestens 98%, mit einer der Sequenzen der SEQ ID NO: 45, 1-10, 50, 23-26, 52, 29-31, 48, 17-19, 51, 27, 28, 53, 32, 33, 54, 34, 35, und 55-58 aufweisen.

Unter dem Begriff "Identität" soll im Zusammenhang mit der vorliegenden Erfindung die Anzahl der übereinstimmenden Nukleotide (Identität), ausgedrückt in Prozent verstanden werden. Bevorzugt wird die Identität zwischen zwei betreffenden Nukleinsäuresequenzen mit Hilfe von Computerprogrammen ermittelt. Weisen Sequenzen, die miteinander verglichen werden, unterschiedliche Längen auf, ist die Identität so zu ermitteln, dass die Anzahl an Nukleotide, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW2 bestimmt. Auf die Definition von Identität in dem Programm ClustalW2 und die Methode zu ihrer Ermittlung, die öffentlich zugänglich sind, wird in dieser Erfindung ausdrücklich Bezug genommen.

ClustalW2 wird öffentlich zur Verfügung gestellt vom European Bioinformatics Institute (EBI) des European Molecular Biology Laboratory (EMBL) und kann im Internet unter http://www.ebi.ac.uk/Tools/msa/clustalw2/ abgerufen werden. Wenn das ClustalW2 Computerprogramm benutzt wird, um die Identität zwischen z.B. der Nukleotidsequenz der im Rahmen der vorliegenden Erfindung beschriebenen Nukleinsäuremoleküle und der Nukleotidsequenz von anderen Nukleinsäuremolekülen zu bestimmen, werden folgende Parameter eingestellt: DNA Weight Matrix: IUB; GAP OPEN: 10; GAP EXTENSION: 0,20; GAP DISTANCES: 5; NO END GAPS: no; ITERATION: none; NUMITER: 1; CLUSTERING: NJ.

Gegenstand der Erfindung ist auch jedes Aptamer, das mit dem komplementären Strang eines zuvor beschriebenen, erfindungsgemäßen Aptamers hybridisiert, vorausgesetzt, dass ein solches Aptamer an Verbindungen aus der Gruppe der Aminoglykoside, insbesondere an eine Verbindung aus der Gruppe der Kanamycine oder Paromomycin, bindet.

Der Begriff "Hybridisierung" bedeutet im Rahmen dieser Erfindung eine Hybridisierung unter stringenten Bedingungen, wie sie in Sambrook et al., Molecular Cloning, A Laboratory Manual, 3. Aufl. (2001) Cold Spring Harbor Laboratory Press, Cold Spring Harbor, NY, beschrieben sind.

Nukleinsäuremoleküle, die mit den genannten Molekülen hybridisieren können beispielsweise aus DNA-Bibliotheken isoliert werden. Die Identifizierung und Isolierung derartiger Nukleinsäuremoleküle kann dabei unter Verwendung der in Anspruch 1 genannten Nukleinsäuremoleküle oder Teile dieser Moleküle bzw. der reversen Komplemente dieser Moleküle erfolgen, z.B. mittels Hybridisierung nach Standardverfahren (siehe z.B. Sambrook et al., 2001, Molecular Cloning, A Laboratory Manual, 3. Aufl. Cold Spring Harbor Laboratory Press, Cold Spring Harbor, NY). Bei als Hybridisierungsprobe verwendeten Fragmenten kann es sich auch um synthetische Fragmente oder Oligonukleotide handeln, die mit Hilfe gängiger Synthesetechniken hergestellt wurden und deren Sequenz im Wesentlichen mit der eines in Rahmen der vorliegenden Erfindung beschriebenen Aptamers bzw. dessen komplementären Strangs übereinstimmt.

Gegenstand der Erfindung ist auch ein Aptamer, das von einem Aptamer mit einer der Sequenzen der SEQ ID NO: 45, 1-10, 50, 23-26, 52, 29-31, 48, 17-19, 51, 27, 28, 53, 32, 33, 54, 34, 35, und 55-58 dadurch abgeleitet ist, dass bei einer dieser ein oder mehrere Nukleotide substituiert, entfernt (deletiert), innerhalb der Sequenz eingefügt (insertiert) und/oder am 5'-Ende und/oder 3'-Ende angefügt sind, wobei ein solches Aptamer an eine oder mehrere Verbindungen aus der Gruppe der Aminoglykoside, insbesondere an eine Verbindung aus der Gruppe der Kanamycine oder Paromomycin, bindet, und unter der Maßgabe, dass ein solches Aptamer eine Identität von mindestens 70% mit der Nukleinsäuresequenz eines Aptamers der SEQ ID NO: 45, 1-10, 50, 23-26, 52, 29-31, 48, 17-19, 51, 27, 28, 53, 32, 33, 54, 34, 35, oder 55-58 aufweist. Derart veränderte Aptamere weisen vorzugsweise eine Identität von mindestens 80%, oder mindestens 85%, oder mindestens 90%, oder mindestens 93%, oder mindestens 95%, oder mindestens 96%, oder mindestens 97%, und am meisten bevorzugt mindestens 98% mit einer der Sequenzen der SEQ ID NO: 45, 1-10, 50, 23-26, 52, 29-31, 48, 17-19, 51, 27, 28, 53, 32, 33, 54, 34, 35, und 55-58 auf. Der Begriff Identität wurde zuvor definiert. Eine Sequenz, bei der ein oder mehr Nukleotide substituiert sind, bezeichnet man auch als Substitutionsmutante, eine Sequenz, bei der ein oder mehr Nukleotide deletiert sind, als Deletionsmutante und eine Sequenz, bei der ein oder mehr Nukleotide insertiert sind, als Insertionsmutante. Vorzugsweise sind insgesamt, bezogen auf Substitution, Deletion und Insertion in einem Aptamermolekül, bis zu 80 Nukleotide substituiert, deletiert, und/oder insertiert, mehr bevorzugt bis zu 60 Nukleotide, oder bis zu 50 Nukleotide, noch mehr bevorzugt bis zu 40 Nukleotide, oder bis zu 30 Nuleotide, insbesondere bevorzugt bis zu 10 Nukleotide oder bis zu 8 Nukleotide oder bis zu 5 Nukleotide, weiterhin bevorzugt bis zu 3 Nukleotide, und am meisten bevorzugt bis zu 2 Nukleotide. Selbstverständlich hängt die Anzahl möglicher Deletionen von der Gesamtzahl Nukleotide in einem Aptamer ab. Bei einem Aptamer mit einer der SEQ ID NO: 55 bis 58 sind bis zu 5, vorzugsweise bis zu 3 Nukleotide, am meisten bevorzugt bis zu 2 Nukleotide substituiert, deletiert und/oder insertiert.

Bei einer Anfügung sind vorzugweise am 5' und/oder am 3' Ende des Aptamers bis zu 100 Nukleotide angefügt, mehr bevorzugt bis zu 50 Nukleotide, noch mehr bevorzugt bis zu 40 Nukleotide, oder bis zu 20 Nuleotide, insbesondere bevorzugt bis zu 10 Nukleotide oder bis zu 8 Nukleotide, am meisten bevorzugt bis zu 5 Nukleotide.

In einer speziellen Ausführungsform, die mit den vorigen kombiniert werden kann, weisen die in den Punkten b) bis f) beschriebenen Aptamer-Abwandlungen, eins oder mehrere der Motive ATACCAGCTTATTCAATT (SEQ ID NO: 59), AGATAGTAAGTGCAATCT (SEQ ID NO: 60) und TGAGGCTCGATC (SEQ ID NO: 61) auf, entweder in unveränderter Form oder in im Wesentlichen unveränderter Form. Vorliegen können somit die oben erwähnten Motive einzeln, oder das Motiv mit der SEQ ID NO: 59 und das Motiv mit der SEQ ID NO: 60, das Motiv mit der SEQ ID NO: 59 und das Motiv mit der SEQ ID NO: 61, das Motiv mit der SEQ ID NO: 60 und das Motiv mit der SEQ ID NO: 61, oder die Motive mit der SEQ ID NO: 59, SEQ ID NO: 60 und SEQ ID NO: 61, entweder in unveränderter Form oder in im Wesentlichen unveränderter Form. Im Wesentlichen unverändert bedeutet, dass bis zu maximal 5 Nukleotide, vorzugsweise maximal 4 Nukleotide, am meisten bevorzugt maximal 3 Nukleotide, bei diesem Motiv substituiert, deletiert und/oder insertiert sind.

Bei einem Aptamer, bei dem gegenüber einem Aptamer, welches eine Nukleinsäuresequenz umfasst, die ausgewählt ist aus einer der SEQ ID NO: 45, 1-10, 50, 23-26, 52, 29-31, 48, 17-19, 51, 27, 28, 53, 32, 33, 54, 34, 35, und 55-58, ein oder mehrere Nukleotide angefügt sind, können Anfügungen am 5'-Ende des Aptamers und/oder am 3'-Ende des Aptamers erfolgen. Bei Anfügungen kann es sich beispielsweise um Oligonukleotide handeln, die als Spacer zwischen der Aptamersequenz und einer Markierung dienen oder um ein Oligonukleotid das eine Sequenz aufweist, die komplementär zu einem gelabelten Oligonukleotid ist, wie beschrieben in WO2005113817. Verschiedene weitere anfügbare Sequenzen, welche die Bindungseigenschaften des Aptamers an sein Target nicht beeinträchtigen, sind dem Fachmann auf dem Gebiet der SELEX Verfahren bekannt, beispielsweise aus Conrad et al., "In Vitro Selection of Nucleic Acid Aptamers That Bind Proteins, "Methods in Enzymology, 267: 336-83 (1996) ; Ciesiolka et al., "Affinity Selection-Amplification from Randomized Ribooligonucleotide Pools, "Methods in Enzymology, 267: 315-35 (1996); and Fitzwater et al., "A SELEX Primer, "Methods in Enzymology, 267: 275-301 (1996).

Fragmente, auch bezeichnet als Teile oder Teilsequenzen, weisen die zuvor beschriebene Funktionalität der erfindungsgemäßen Aptamere auf. Fragmente werden erhalten, indem man am 5'-Ende und/oder am 3'-Ende eines erfindungsgemäßen Aptamers eine oder mehrere Nukleinsäuren entfernt. Fragmente haben vorzugsweise eine Länge von mindestens 10, insbesondere von mindestens 15 und besonders bevorzugt von mindestens 20 Nukleotiden, mehr bevorzugt mindestens 30 Nukleotiden, noch mehr bevorzugt mindestens 40 Nukleotiden, weiterhin bevorzugt mindestens 50 Nukleotiden, und am meisten bevorzugt mindestens 60 Nukleotiden. Fragmente sind beispielsweise solche Aptamere, bei welchen die Motive ATACCAGCTTATTCAATT (SEQ ID NO: 59) und AGATAGTAAGTGCAATCT (SEQ ID NO: 60) ganz oder teilweise entfernt sind.

Der Begriff "Derivat" bezeichnet im Zusammenhang mit der vorliegenden Erfindung ein Aptamer, das eine chemische Struktur aufweist, die in natürlicher DNA oder RNA nicht vorkommt.

Insbesondere bezeichnet der Begriff Derivat ein Aptamer, das eine chemische Struktur enthält, die von Desoxyribose, Ribose, Phosphat, Adenin (A), Guanin (G), Cytosin (C), Thymin (T), oder Uracil (U) abweichend ist. Ein Aptamer-Derivat kann an der Nukleobase, an der Pentose oder am Phosphat-Rückgrat modifiziert sein.

Insbesondere bezeichnet der Begriff "Derivat" ein Aptamer,
- bei dem natürlich in DNA und RNA vorkommende Nukleotide teilweise oder vollständig durch chemisch davon abweichende Nukleotide, sogenannte modifizierte Nukleotide, ersetzt sind und/oder
- dessen molekulare Struktur anderweitig modifiziert ist, insbesondere durch Anbindung nicht natürlich in RNA oder DNA vorkommender Strukturen, und/oder
- die ein modifiziertes Rückgrat aufweisen

Spezielle Beispiele für Derivate sind, ohne darauf beschränkt zu sein,
- Aptamere, die an mindestens einem Nukleotid eine Alkylierung, Arylierung oder Acetylierung, Alkoxylierung, Halogenierung, eine Aminogruppe oder eine andere funktionelle Gruppe aufweisen. Beispiele für modifizierte Nukleotide sind 2'-Fluor-Ribonucleotide, 2'-NH₂-, 2'-OCH₃- und 2'-O-methoxyethyl-Ribonukleotide, die für RNA-Aptamere genutzt werden.
- Aptamere, die eine Basenmodifikation aufweisen, wie Bromuridin,
- Markierte Aptamere, auch bezeichnet als gelabelte Aptamere. Bevorzugte Markierungen (Label) sind visuell, optisch, photonisch, elektronisch, akustisch, opto-akustisch, nach Masse, elektrochemisch, elektrooptisch, spektrometrisch, enzymatisch, oder anderweitig chemisch, biochemisch oder physikalisch detektierbar. Label können beispielsweise angebundene Reporter-, Marker- oder Adaptermoleküle sein. Beispiele hierfür sind markierte Aptamere, deren Markierung durch Lumineszenz, UV/VIS-Farbgebung, enzymatisch, elektrochemisch, immunologisch oder radioaktiv nachgewiesen werden kann. Beispiele für Markierungssubstanzen sind weiter unten bei der Erläuterung von erfindungsgemäßen Verfahren angegeben, bei denen markierte Aptamere eingesetzt werden können.
- Aptamere, die enantiomere Nukleotide aufweisen.
- Aptamere, die ganz oder teilweise als Phosphorthioat-RNA oder -DNA, Phosphordithioat-RNA oder -DNA, Phosphorselenoat-RNA oder -DNA, Phosphordiselenoat-RNA oder -DNA, Phosphoroamidat-RNA oder -DNA, locked nucleic acid (LNA), peptide nucleic acid (PNA), N3'-P5' Phosphoramidat RNA/DNA, Cyclohexennukleinsäure (CeNA), Tricyclo-DNA (tcDNA) oder Spiegelmer vorliegen, oder die Phosphoramidatmorpholin (PMO) Komponenten aufweisen (siehe auch Chan et al., Clinical and Experimental Pharmacology and Physiology (2006) 33, 533-540).

Durch manche der Modifikationen können Aptamere gegen Nukleinsäure-spaltende Enzyme stabilisiert werden. Bei der Stabilisierung der Aptamere kann grundsätzlich zwischen der nachträglichen Modifikation der Aptamere und der Selektion mit bereits modifizierter RNA/DNA unterschieden werden. Die Stabilisierung berührt die Affinität der modifizierten RNA/DNA-Aptamere nicht, verhindert jedoch die schnelle Zersetzung der Aptamere in einem Organismus oder biologischen Lösungen durch RNasen/DNasen. Ein Aptamer wird im Rahmen der Erfindung als stabilisiert bezeichnet, wenn die Halbwertszeit in biologischen Seren grösser als eine Minute, vorzugsweise grösser als eine Stunde, besonders bevorzugt grösser als ein Tag ist. Die Aptamere können auch mit Reportermolekülen modifiziert sein, die neben der Detektion der markierten Aptamere auch zur Erhöhung der Stabilität beitragen können.

Zu den Punkten b) -f) gelten, auch für die noch folgenden Ausführungsformen, analog die weiter oben ausgeführten Offenbarungen.

Bei den obigen, Aptamere mit der SEQ ID NO: 45, 50 oder 52, betreffenden Ausführungsformen, weisen die in den Punkten b) bis f) beschriebenen Aptamer-Abwandlungen eins oder mehrere der Motive ATACCAGCTTATTCAATT (SEQ ID NO: 59), AGATAGTAAGTGCAATCT (SEQ ID NO: 60) und GKATX₄X₂GGCTYGRTY (SEQ ID NO: 58) auf. X₂ bedeutet entweder A oder L, und X₄ bedeutet G, A oder L, wobei L für kein Nukleotid (Deletion) steht) auf, wobei die Motive in unveränderter Form oder in im Wesentlichen unveränderter Form vorliegen können. Vorliegen können somit die oben erwähnten Motive einzeln, oder das Motiv mit der SEQ ID NO: 58 und das Motiv mit der SEQ ID NO: 59, das Motiv mit der SEQ ID NO: 58 und das Motiv mit der SEQ ID NO: 60, das Motiv mit der SEQ ID NO: 59 und das Motiv mit der SEQ ID NO: 60, oder die Motive mit der SEQ ID NO: 58, SEQ ID NO: 59 und SEQ ID NO: 60, entweder in unveränderter Form oder in im Wesentlichen unveränderter Form. Im Wesentlichen unverändert bedeutet, dass bis zu maximal 5 Nukleotide, vorzugsweise maximal 4 Nukleotide, am meisten bevorzugt maximal 3 Nukleotide, bei diesem Motiv substituiert, deletiert und/oder insertiert sind.

Eine spezielle Ausführungsform der Erfindung betrifft ein Aptamer, das an eine oder mehrere Verbindungen aus der Gruppe der Aminoglykoside, insbesondere an eine Verbindung aus der Gruppe der Kanamycine oder Paromomycin, bindet, und das ausgewählt ist aus der Gruppe bestehend aus
a) einem Aptamer umfassend, oder bestehend aus, eine(r) Nukleinsäuresequenz, die ausgewählt ist aus der Gruppe bestehend aus der Gruppe bestehend aus SEQ ID NO: 45, 1-10, 50, 23-26, 52, 29-31, 48, 17-19, 51, 27, 28, 53, 32, 33, 54, 34, 35, wobei am 5'-Ende der Nukleinsäuresequenz der SEQ ID NO: 45, 1-10, 50, 23-26, 52, 29-31, 48, 17-19, 51, 27, 28, 53, 32, 33, 54, 34, oder 35 ein Oligonukleotid mit der Sequenz 5' ATACCAGCTTATTCAATT 3' (SEQ ID NO: 59) deletiert ist und/oder am 3'-Ende der Nukleinsäuresequenz der SEQ ID NO: 45, 1-10, 50, 23-26, 52, 29-31, 48, 17-19, 51, 27, 28, 53, 32, 33, 54, 34, oder 35 ein Oligonukleotid mit der Sequenz 5' AGATAGTAAGTGCAATCT 3' (SEQ ID NO: 60) deletiert ist, mit der Maßgabe, dass Thymin durch Uracil ersetzt sein kann,
b) einem Aptamer, dessen Nukleinsäuresequenz eine Identität von mindestens 70% mit der Nukleinsäuresequenz eines Aptamers aus a) aufweist
c) einem Aptamer, das mit dem komplementären Strang eines Aptamers aus a) unter stringenten Bedingungen hybridisiert,
d) einem Aptamer bei dem gegenüber einem Aptamer aus a) ein oder mehrere Nukleotide substituiert, deletiert, insertiert und/oder angefügt sind, wobei dieses Aptamer eine Identität von mindestens 70% mit der Nukleinsäuresequenz eines Aptamers aus a) aufweist,
e) einem Fragment eines Aptamers nach a), b), c) oder d), und
f) einem Derivat eines Aptamers nach a), b), c), d) oder e), das eine chemische Struktur aufweist, die in natürlicher DNA oder RNA nicht vorkommt.

Die Bindungsfähigkeit der erfindungsgemäßen Aptamere wird durch die Affinität (Sensitivität) der Bindung (ausgedrückt durch die Dissoziationskonstante) beschrieben. In einem weiteren Aspekt betrifft die Erfindung ein Verfahren zum Nachweis einer oder mehrerer Verbindungen aus der Gruppe der Aminoglykoside, insbesondere einer oder mehrerer Verbindungen aus der Gruppe der Kanamycine oder Paromomycin, bei dem
a) ein oder mehrere verschiedene Aptamere wie zuvor beschrieben mit einer Probe, die eine oder mehrere Verbindungen aus der Gruppe der Aminoglykoside enthält, in Kontakt gebracht wird/werden und
b) eine Bindung zwischen dem/den Aptamer(en) und der/den Verbindung(en) aus der Gruppe der Aminoglykoside nachgewiesen wird.

Das Verfahren kann sowohl ein qualitatives als auch ein quantitatives Nachweisverfahren sein. Die Verfahrensschritte a) und b) können je nach konkreter Verfahrensgestaltung gleichzeitig oder im Wesentlichen gleichzeitig erfolgen.

Das Verfahren ist insbesondere zum Einsatz in der Lebensmittel-, Wasser- und Umweltanalytik, der Wasserbehandlung und der Wasseraufbereitung, insbesondere von Trinkwasser und Abwasser, der Diagnostik sowie zur Anwendung in Krankenhäusern und Pflegeeinrichtungen geeignet.

Das Verfahren eignet sich insbesondere zum Nachweis von einer Verbindung aus der Gruppe der Aminoglykoside, insbesondere der Kanamycine oder Paromomycin, insbesondere Kanamycin A, B oder C, in beliebigen Medien und Umgebungen, insbesondere in Wasser und anderen Flüssigkeiten, wie beispielsweise in Trink- und Abwasserproben.

Eine Probe im Sinne des obigen Nachweisverfahrens ist ein bereitgestelltes oder durch Probenentnahme erhaltenes Material, von dem angenommen wird, dass es eine oder mehrere Verbindungen aus der Gruppe der Aminoglykoside, insbesondere eine oder mehrere Verbindungen aus der Gruppe der Kanamycine oder Paromomycin, (zusammenfassend bezeichnet als "Target" oder im Rahmen dieses Nachweisverfahrens auch als "Analyt") umfasst, und das auf das Vorhandensein von Target geprüft werden soll.

Eine Probe kann eine Wasserprobe, insbesondere eine Trinkwasser-, Grundwasser-, Oberflächenwasser- oder Abwasserprobe, eine Probe von anderweitigem, aus der Umwelt entnommenem Material, eine klinische Probe oder eine Lebensmittelprobe sein.

Eine Probe können weiterhin alle biologischen Materialien sein, die von Individuen isoliert worden sind, beispielsweise biologische Gewebe und Flüssigkeiten, zu denen u.a. Blut, Haut, Plasma, Serum, Lymphe, Urin, Gehirnflüssigkeit, Tränen, Abstriche, Gewebeproben, Organe und Tumore zählen. Vorliegend sind in Proben auch Bestandteile von Zellkulturen eingeschlossen. Die Probenentnahme erfolgt insbesondere so, dass die entnommene Teilmenge einem Durchschnitt der gesamten Menge entspricht. Die durch Untersuchung der Probe ermittelten Merkmale dienen der Beurteilung der durch die Probe erfassten Menge, die wiederum Rückschlüsse auf die Gesamtmenge, beispielsweise das Blut bzw. die Lymphe in einem Organismus, zulässt. Für die Untersuchung können die Proben vorbehandelt, wie z.B. durch Mischen, Zugabe von Enzymen oder Markern, oder aufgereinigt werden.

Wird das Aptamer mit dem Target in Kontakt gebracht, so bildet sich ein Aptamer-Target-Komplex durch Bindung des Aptamers an das Target. Die Bindung bzw. das Bindungsereignis kann beispielsweise visuell, optisch, photonisch, elektronisch, akustisch, opto-akustisch, nach Masse, elektrochemisch, elektrooptisch, spektrometrisch, enzymatisch, oder anderweitig chemisch, biochemisch oder physikalisch nachgewiesen werden.

Bei einem markierungsfreien Nachweis wird das Aptamer beispielsweise auf einer Oberfläche fixiert und die Änderung der Schichtdicke nach Anlagerung des Targets mit einer der genannten Methoden bestimmt, wie z.B. über eine Änderung der optischen Eigenschaften der Sensorschicht (z.B. Brechungsindex). Ein weiteres Verfahren des markierungsfreien Nachweises ist die Messung des Masseänderung nach Bindung des Aptamers an das Target mit einer Mikrowaage, oder die Messung einer Frequenzänderung eines Schwingquarzes nach Bindung des Aptamers an das Target, welches auf der Oberfläche des Schwingquarzes bereitgestellt wird.

In Fällen, in denen die Komplexierung nicht direkt detektierbar ist, kann der Komplex durch eine Markierung sichtbar gemacht werden, beispielsweise in einer Indikatorreaktion nach einer direkten oder indirekten Kopplung eines Komplexpartners mit einer Markierungssubstanz. Entweder kann das verwendete Aptamer oder das Target mit einer Markierung (Label) versehen sein. Bevorzugt ist das Aptamer markiert.

Bevorzugte Markierungen (Label) sind visuell, optisch, photonisch, elektronisch, akustisch, opto-akustisch, nach Masse, elektrochemisch, elektrooptisch, spektrometrisch, enzymatisch, oder anderweitig physikalisch, chemisch oder biochemisch detektierbar. In einer Ausführungsform des Verfahrens wird die Markierung durch Lumineszenz, UV/VIS-Spektroskopie, enzymatisch, elektrochemisch oder radioaktiv nachgewiesen.

Lumineszenz betrifft die Emission von Licht. Im erfindungsgemäßen Verfahren finden beispielsweise die Photolumineszenz, die Chemilumineszenz und die Biolumineszenz zum Nachweis der Markierung Anwendung. Bei der Photolumineszenz oder Fluoreszenz erfolgt die Anregung durch Absorption von Photonen. Beispielhafte Fluorophore sind, ohne Beschränkung, Bisbenzimidazol, Fluoreszein, Acridinorange, Cy5, Cy3 oder Propidiumiodid, die kovalent an Aptamere gekoppelt werden können, Tetramethyl-6-Carboxyrhodamin (TAMRA), Texas Red (TR), Rhodamin, Alexa Fluor Farbstoffe (u.a. Fluoreszenzfarbstoffe verschiedener Wellenlängen von verschiedenen Firmen). Die Auswertung geschieht visuell oder mit entsprechenden Messgeräten, z.B. im Multilabel Counter, im Fluoreszenzmikroskop, oder durch Durchflusszytometrie, z.B. im Cytofluorimeter. Chemilumineszenz beschreibt die Emission sichtbaren Lichts als Folge einer chemischen Reaktion. Biolumineszenz bezeichnet die Emission sichtbaren Lichts als Folge einer Enzymreaktion, beispielsweise einer durch das Enzym Luciferase katalysierten Redoxreaktion.

Andere Markierungsstoffe sind Katalysatoren, kolloidale metallische Teilchen, z.B. Gold-Nanopartikel, kolloidale nicht metallische Teilchen, Quantum Dots, organische Polymere, Latexteilchen, oder Liposome mit signalerzeugenden Stoffen. Kolloidale Teilchen können kolorimetrisch nachgewiesen werden.

Auch einsetzbar sind visuell detektierbare Farbstoffe, wie beispielsweise interkalierende Farbstoffe.

Einsetzbar als Marker sind auch Enzyme, deren enzymatische Reaktion durch den Verbrauch oder die Entstehung detektierbarer Substrate oder Produkte gekennzeichnet ist, wobei ohne Beschränkung eine optische oder elektrochemische Detektion angewandt werden kann. Ein Nachweis kann z.B. mit Enzymen als Markierungssubstanzen geführt werden, die Substrate zu farbigen Produkten umsetzen, vorzugsweise Peroxidase, Green Fluorescent Protein (GFP), Luciferase, [beta]-Galactosidase oder Alkalische Phosphatase. Beispielsweise wird das farblose Substrat X-Gal durch die Aktivität der [beta]-Galactosidase zu einem blauen Produkt umgesetzt, dessen Farbgebung visuell erfasst wird.

Ein oben genanntes enzymatisches Marker- und Nachweissystem verwendet Alkalische Phosphatase. Mit Alkalischer Phosphatase (APP) sind verschiedene Nachweise möglich, wie nachfolgend beispielhaft dargestellt:
- elektrochemischer Nachweis: Substrat Phenylphosphat, enzymatische Reaktion von APP bildet Phenol, wird an Phenolsensor (z.B. mit immobilisierter Tyrosinase) elektrochemisch detektiert
- Messung der Farbreaktion: Substrat p-Nitrophenylphosphat, enzymatische Reaktion von APP bildet p-Nitrophenol, ist gelb gefärbt
- Fluoreszenz-Nachweis: Substrat 4-Methylumbelliferonylphosphat: enzymatische Reaktion von APP bildet Methylumbelliferonyl-Rest, der nach Anregung Fluoreszenz freisetzt
- für Chemilumineszenz-Nachweis: Substrat AMPPD (3-(2'-Spiroadamantan)-4-Methoxy-4-(3"-Phosphoryloxy)phenyl-1,2-Dioxetan): enzymatische Reaktion von APP bildet AMP-D und setzt hv (Chemilumineszenz) frei

Ferner setzt APP 5-Brom-4-Chlor-3-Indolylphosphat (BCIP oder X-Phosphat) und Nitroblau-Tetrazoliumsalz (NBT) farbig um. Die Farbstoffe fallen dabei in unmittelbarer Nähe der AP-Moleküle aus und färben die Umgebung der gebundenen Verbindungen dunkelviolett an.

Die Peroxidase katalysiert z.B. die Oxidation von ABTS (2,2'-Azino-bis-[3-ethylbenzthiazolin-6-sulfonsäure]) in Gegenwart von H₂O₂. Aufgrund der Enzymstabilität und einer Vielzahl an möglichen Substraten ist die Meerrettich-Peroxidase bevorzugt. Weitere Enymmarker, die die Erzeugung detektierbarer Produkte katalysieren sind Chloramphenicolacetyltransferase (CAT) und Glutathion-S-Transferase (GST).

Der Nachweis kann auch mittels radioaktiver Isotope erfolgen, mit denen das Aptamer markiert ist, vorzugsweise 3H, 14C, 32P, 33P, 35S oder 1251, besonders bevorzugt 32P, 33P oder 1251. Bei der Szintillationszählung wird die vom radioaktiv-markierten Aptamer-Target-Komplex abgegebene radioaktive Strahlung indirekt gemessen. Eine Szintillatorsubstanz wird durch die radioaktive Strahlung angeregt. Beim Übergang in den Grundzustand wird die Anregungsenergie wieder als Lichtblitze freigesetzt, welche durch einen Photoelektronenvervielfacher (Photomultiplier) verstärkt und gezählt werden.

Die Aptamere können auch mit Digoxigenin oder Biotin markiert sein, die beispielsweise durch Antikörper oder Streptavidin gebunden werden, die wiederum eine Markierung tragen können, wie z.B. ein Enzymkonjugat. Die vorherige kovalente Verknüpfung (Konjugation) eines Antikörpers mit einem Enzym kann auf verschiedene bekannte Arten erfolgen. Der Nachweis der Antikörper-Bindung kann auch radioaktiv in einem RIA (radioactive immunoassay) mit radioaktiven Isotopen, vorzugsweise mit 125I, oder durch Fluoreszenz in einem FIA (Fluoroimmunoassay) mit Fluorophoren, vorzugsweise mit Fluorescein oder FITC, erfolgen.

Die genannten Methoden schließen vorzugsweise Waschschritte ein, um ungebundene und/oder unspezifisch gebundene Aptamere und/oder Nachweisreagenzien abzutrennen. Die Durchführung sämtlicher Nachweisverfahren ist dem Fachmann bekannt. Der direkte Nachweis der Markierung ist im vorliegenden Verfahren der Erfindung bevorzugt, insbesondere der direkte Nachweis durch Fluoreszenz.

In einer weiteren Ausgestaltung des Verfahrens wird der Nachweis in-situ durchgeführt. Diese Umsetzung erfordert einen geeigneten Inkubationsraum, auf dem der Nachweis einfach durchgeführt und sichtbar gemacht werden kann. Hierzu wird vorteilhaft ein fester Träger verwendet, der es ermöglicht, Probe, Aptamere und ggf. Nachweisreagenzien der Komplexe zu fixieren. Die Aptamere können sowohl vor oder nach der Probe auf den festen Träger aufgebracht werden. Methoden der Immobilisierung vorgenannter Aptamere sind dem Fachmann bekannt. Beispiele sind weiter unten angegeben. Für den Nachweis kommen alle Nachweise in Betracht, die zuvor bereits genannt sind, wie markierungsfreie Nachweise und Nachweise mit Verwendung einer Markierung. Bei einem Farbnachweis können die Aptamere so markiert sein, so dass direkt im Anschluss an die Inkubation ein Ablesen und Auswerten realisiert werden kann.

In den zuvor erläuterten Verfahren und allen anderen von dieser Erfindung offenbarten Verfahren kann das erfindungsgemäße Aptamer allein, oder eine Mischung verschiedener erfindungsgemäßer Aptamere eingesetzt werden. Erfindungsgemäße Aptamere können auch in Kombination mit anderen Aptameren angewendet werden. Eine Kombination mit anderen Aptameren für andere Targets (d.h. andere Targets als eine Verbindung aus der Gruppe der Aminoglykoside) bietet den Vorteil, dass ein entsprechendes Verfahren gleichzeitig auch zum Nachweis, Anreicherung, Abtrennung und/oder Isolierung anderer Targets anwendbar ist.

Wie bereits erwähnt, können die Probe oder das Aptamer an eine feste Phase immobilisiert sein. In dem Verfahren können beispielsweise (Micro)-Arrays des erfindungsgemäßen Aptamers, oder Mikrotiterplatten-Testsysteme eingesetzt werden. Aptamere binden an das Target ähnlich stark wie Antikörper an ihr Target (Antigen). Daher ist das oben beschriebene Nachweisverfahren analog durchführbar wie bekannte Immunoassays, wobei im Vergleich zu einem bekannten Immunoassay einer oder mehrere Antikörper durch ein erfindungsgemäßes Aptamer ersetzt sind.

Beispielsweise ist es möglich, das Nachweisverfahrens in Form eines kompetitiven Assays oder eines Sandwich-Assays durchzuführen.

Bei einer Ausführungsform eines kompetitiven Assays wird ein für das Target spezifisches erfindungsgemäßes Aptamer an eine feste Phase gebunden. Anschließend erfolgt die Zugabe der Probenlösung, die das zu messende Target enthält und gleichzeitig mit einer bekannten Konzentration an markiertem Target versetzt ist. Sowohl das in der Probenlösung in unbekannter Konzentration vorliegende unmarkierte Target als auch das markierte Target konkurrieren um die Bindung an das gebundene Aptamer. Je höher die Konzentration des Targets in der Probe ist, desto weniger markiertes Target wird demzufolge an das Aptamer gebunden. Über die Erkennung und gegebenenfalls eine quantitative Bestimmung der Markierung sind der Nachweis des Targets in der Probe und die Messung seiner Konzentration möglich.

In einer anderen Ausführungsform eines kompetetiven Assays wird ebenfalls zunächst ein für das Target spezifisches erfindungsgemäßes Aptamer an eine feste Phase gebunden. Daran wird markiertes Target gebunden. Die Messung der Markierung ergibt das Ausgangssignal. Die Zugabe von Probe mit unmarkiertem Target verdrängt gebundenes markiertes Target, und das abnehmende Messsignal ist der Probenkonzentration proportional.

Bei einem klassischen immunologischen Sandwich-Assay werden mindestens zwei Antikörper eingesetzt. Zunächst wird einer der beiden Antikörper an eine feste Phase immobilisiert. Dieser wird als Primärantikörper oder auch als Fänger bezeichnet. Nach der Zugabe der Probe bindet das darin enthaltene Antigen an den Primärantikörper. Anschließend wird die Probenlösung entfernt und der als Sekundärantikörper oder Detektor bezeichnete zweite Antikörper in gelöster Form auf die feste Phase gegeben. Der Detektor bindet nun ebenfalls an das durch den Primärantikörper gebundene Antigen. Für den Nachweis und die Quantifizierung ist der Sekundärantikörper entweder selbst markiert, oder er wird über ein markiertes Reagens nachgewiesen. In der vorliegenden Erfindung wird dieses Verfahren dahin gehend abgewandelt, dass entweder der Primärantikörper oder der Sekundärantikörper, oder beide, durch ein erfindungsgemäßes Aptamer ersetzt werden und dass das Antigen ein Target für das Aptamer, also eine Verbindung aus der Gruppe der Aminoglykoside, insbesondere eine Verbindung aus der Gruppe der Kanamycine oder Paromomycin, ist. Der Assay kann mit allen bekannten festen Phasen durchgeführt werden, beispielsweise Mikrotiterplatten, Streifen, Membranen, Küvetten etc.

Die Erfindung stellt somit in einer speziellen Ausführungsform ein Verfahren zum Nachweis von Verbindungen aus der Gruppe der Aminoglykoside, insbesondere einer Verbindung aus der Gruppe der Kanamycine oder Paromomycin, zur Verfügung, bei dem
a) ein erster Antikörper oder ein erfindungsgemäßes Aptamer, der/das für eine Verbindung aus der Gruppe der Aminoglykoside spezifisch ist, an eine feste Phase immobilisiert wird,
b) die feste Phase mit daran immobilisiertem Antikörper oder Aptamer mit einer Probe, die eine Verbindung aus der Gruppe der Aminoglykoside enthält, inkubiert wird, und
c) die feste Phase mit einem zweiten Antikörper oder einem erfindungsgemäßen Aptamer, der/das für eine Verbindung aus der Gruppe der Aminoglykoside spezifisch ist, inkubiert wird, und
d) eine Bindung zwischen dem zweiten Antikörper oder dem Aptamer und der Verbindung aus der Gruppe der Aminoglykoside nachgewiesen wird, unter der Bedingung, dass mindestens in einem der Schritte a) oder c) ein erfindungsgemäßes Aptamer statt eines Antikörpers eingesetzt wird.

Die Immobilisierung in Schritt a) kann mit allen üblichen Techniken erfolgen, die auch aus immunologischen Assays bekannt sind. Der Nachweis in Schritt d) kann im Falle eines Antikörpers durch die bekannten Methoden erfolgen, die aus immunologischen direkten und indirekten Sandwich-Assays bekannt sind. Entweder kann der Antikörper selbst markiert sein, beispielsweise durch Anbindung eines Enzyms, das eine Farbreaktion zum Nachweis katalysieren kann, oder es kann ein dritter Antikörper zugegeben werden, der seinerseits an den zweiten Antikörper bindet und eine Markierung aufweist. Sofern in Schritt c) ein Aptamer eingesetzt wird, ist dieses vorzugsweise ein markiertes Aptamer, das mit geeigneten Nachweisreaktionen detektiert werden kann, wie zuvor bereits beschrieben. Zwischen den genannten Schritten a) -d) werden vorzugsweise Waschschritte durchgeführt, z.B. mit üblichen und bekannten Waschpuffern. Der Begriff Immobilierung bedeutet bei diesem Verfahren, dass der in Schritt a) immobilisierte Antikörper oder das Aptamer nicht durch einen Waschschritt von der festen Phase entfernt wird.

In einem weiteren Aspekt betrifft die Erfindung ein Verfahren zur Anreicherung, Abtrennung und/oder Isolierung einer oder mehrerer Verbindungen aus der Gruppe der Aminoglykoside, insbesondere einer oder mehrerer Verbindungen aus der Gruppe der Kanamycine oder Paromomycin, bei dem
a) ein oder mehrere verschiedene Aptamere wie zuvor beschrieben mit einer Probe, die eine oder mehrere Verbindungen aus der Gruppe der Aminoglykoside enthält, in Kontakt gebracht wird/werden, wobei ein Komplex oder Komplexe aus dem/den Aptamer(en) und der/den Verbindung(en) aus der Gruppe der Aminoglykoside gebildet wird,
b) der/die Komplex(e) und die übrige Probe voneinander getrennt werden, und
c) optional die Verbindung(en) aus der Gruppe der Aminoglykoside aus dem Komplex isoliert wird/werden.

Eine "Probe" im Sinne dieses Verfahrens ist ebenso definiert wie eine Probe im Sinne des zuvor beschriebenen Nachweisverfahrens. Eine Probe ist insbesondere eine Wasserprobe, wie zum Beispiel eine Trinkwasser-, Grundwasser-, Oberflächenwasser- oder Abwasserprobe, eine Probe von anderweitigem aus der Umwelt entnommenem Material, oder eine Lebensmittelprobe.

Auch dieses Verfahren kommt insbesondere zum Einsatz in der Lebensmittel-, Wasser- und Umweltanalytik, der Wasserbehandlung und der Wasseraufbereitung, insbesondere von Trinkwasser und Abwasser, der Diagnostik sowie zur Anwendung in Krankenhäusern und Pflegeeinrichtungen.

Eine "Abtrennung" von einer oder mehrerer Verbindungen aus der Gruppe der Aminoglykoside, insbesondere einer oder mehrerer Verbindungen aus der Gruppe der Kanamycine oder Paromomycin (zusammengefasst bezeichnet als "Target") aus einer Probe kann, im Rahmen der analytischen Nachweisgrenze, vollständig sein oder nicht vollständig sein. Eine vollständige Abtrennung wird hierin auch als "Entfernung" bezeichnet.

Eine "Anreicherung" wird in Schritt b) erzielt, wenn der gebildete Aptamer-Target-Komplex und die übrige Probe voneinander getrennt werden. Es werden zwei Fraktionen erhalten, wobei in der Fraktion, die den Komplex enthält, der Komplex angereichert ist. Der Begriff "Anreicherung" kann sich auf den Komplex beziehen, der aus Aptamer/en und einer oder mehrerer Verbindungen aus der Gruppe der Aminoglykoside gebildet ist, oder auf die Verbindung/en aus der Gruppe der Aminoglykoside selbst, d.h. das Target ohne Aptamer. Zur Anreicherung des Targets kann der Verfahrensschritt c) durchgeführt werden. Eine Anreicherung bedeutet mit anderen Worten eine Erhöhung der Konzentration des Aptamer-Target-Komplexes oder des Targets selbst.

Der Begriff "Isolierung" kann sich auf den Komplex beziehen, der aus Aptamer/en und einer oder mehrerer Verbindungen aus der Gruppe der Aminoglykoside gebildet ist, oder auf die Verbindung/en aus der Gruppe der Aminoglykoside selbst. Daher ist der letzte Verfahrensschritt optional.

Die oben beschriebene Abtrennung, Anreicherung, und Isolierung können mit dem Verfahren gleichzeitig verwirklicht werden. Vorzugsweise wird das Verfahren aber zu einem dieser Zwecke durchgeführt.

Der Begriff "Komplex" bezeichnet die Struktur, die bei der Bindung des Aptamers an sein Target gebildet wird. Somit bezeichnet der Begriff "Komplex" eine Aptamer-Target-Struktur, bei der das Aptamer an das Target gebunden ist. Wie einleitend erläutert, erfolgt die Aptamer-Target-Bindung vorwiegend, aber nicht unbedingt ausschließlich, über die Strukturkompatibilität, so genannte "Stacking interactions" bei aromatischen Ringstrukturen (Stapelkräfte durch Elektronenwechselwirkung mit Nachbarbasen), elektrostatische Wechselwirkungen (z.B. Van der Waals-, Ionen-, Dipolkräfte) und Wasserstoffbrückenbindungen.

Die genannten Verfahrensschritte a), b) und c) können je nach Verfahrensgestaltung gleichzeitig erfolgen, und insbesondere können die Verfahrensschritte a) und b) gleichzeitig erfolgen.

Bei der Kontaktierung von Target und Aptamer kann die Bildung des Aptamer-Target-Komplexes durch Temperierung auf die optimale Aptamer-Target-Bindungstemperatur, bevorzugt 20 - 25°C, und/oder Rühren der Probe befördert werden. Nach einer Inkubationszeit werden die Aptamer-Target-Komplexe, von der restlichen Probenlösung abgetrennt. Geeignete Methoden sind dem Fachmann bekannt, wie z.B. Dialyse, Ultrafiltration, Gelfiltration, Chromatographie, magnetische Trennung oder Waschschritte, sofern der Komplex immobilisiert vorliegt.

Durch Veränderungen der für die Aptamerbindung erforderlichen definierten Bedingungen wird im optionalen Schritt c) der Aptamer-Target-Komplex getrennt. Hierzu sind physikochemische Einwirkungen, wie z.B. eine Variation von Salzkonzentration oder pH oder eine hitzebedingte Denaturierung, denkbar. Abschließend ist eine weitere Reinigung des Targets möglich, wobei das Aptamer zuvor abgebaut werden kann, z.B. durch saure (thermische) Hydrolyse oder DNasen. Sofern das Aptamer immobilisiert ist, kann das Target eluiert werden, wie z.B. von einer Säule, die anschließend regeneriert und wiederverwendet wird.

Erfindungsgemäß können die Aptamere auch an einer festen Phase, genauer gesagt an der Oberfläche einer festen Phase, immobilisiert sein, vorzugsweise über ein Spacer-Molekül, das beispielsweise eine Linker-Nukleinsäure sein kann. Geeignete Methoden der Immobilisierung sind dem Fachmann bekannt, die sowohl mit einer kovalenten Kopplung als auch einer nicht-kovalenten Kopplung mittels geeigneter Affinitätspaare, wie z.B. Biotin/Streptavidin, einhergehen können. Die feste Phase können beispielsweise Platten, Streifen, Membranen, Filme, Gele, Perlen, Mikro- und Nanopartikel sein. Beispielhafte Trägermaterialien sind anorganische und organische Polymere, Keramiken, Gläser, Metalle, insbesondere Edelmetalle. Hierzu zählen Kunststoffe, beispielsweise auf Basis von Polystyrol. Des Weiteren sind Biopolymere, vorzugsweise Cellulose, Dextran, Agar, Agarose und Sephadex, die funktionalisiert sein können, insbesondere als Nitrocellulose oder Cyanbromid-Sephadex, als Polymere im erfindungsgemäßen Verfahren einsetzbar. Aptamere können an Magnetic Beads gebunden werden, deren Oberfläche z.B. mit Tosyl- oder Epoxy-Gruppen, mit Amino- oder Carboxyl-Gruppen oder mit Streptavidin funktionalisiert sind. Weiterhin ist eine Kopplung von Magnetic Beads an die Desoxyribose der Aptamere, wie z.B. über eine Hydrazon-Bindung, möglich. Die Beispiele für Polymere sind nicht limitierend und andere sind denkbar. Bevorzugte Kombinationen von geometrischer Form und Material sind Gele aus Biopolymeren, insbesondere Agarose, und Gelmatrices aus Dextran und Sephadex, die - beispielsweise in Säulen verpackt - Hohlräume definierter Porengröße ausbilden. In einer speziellen Ausgestaltung ist das soeben beschriebene Abtrennungs-, Anreicherungs-, und/oder Isolierungsverfahren ein Affinitätschromatographie-Verfahren, bei dem ein erfindungsgemäßes Aptamer an einer festen Phase immobilisiert ist, vorzugsweise an Kügelchen (Beads) oder einem anderweitigen chromatographischen Säulenmaterial, wobei alle geometrische Formen einer festen Phase verwendbar und alle oben genannten Substanzen beispielhaft einsetzbar sind.

Mit Aptamer-modifizierten Oberflächen können die entsprechenden Targets ausgehend von geringen Konzentrationen angereichert werden.

Die Erfindung betrifft auch die Verwendung des zuvor beschriebenen Aptamers zum Nachweis, Anreicherung, Abtrennung und/oder Isolierung einer oder mehrerer Verbindungen aus der Gruppe der Aminoglykoside, insbesondere einer oder mehrerer Verbindungen aus der Gruppe der Kanamycine oder Paromomycin. Insbesondere kann das Aptamer in den zuvor erläuterten Verfahren eingesetzt werden. Der Begriff Anreicherung schließt die Aufreinigung des Targets mit ein.

In einem weiteren Aspekt betrifft die Erfindung auch eine Aptamersonde zur Detektion einer oder mehrerer Verbindungen aus der Gruppe der Aminoglykoside, insbesondere einer oder mehrerer Verbindungen aus der Gruppe der Kanamycine oder Paromomycin, wobei die Sonde ein erfindungsgemäßes Aptamer und ein Markierungsmittel (Label) umfasst. Das Markierungsmittel kann auf verschiedene Art und Weise an das Aptamer gebunden sein, beispielsweise, und nicht beschränkend, durch kovalente Bindung, Komplexierung, DNA/RNA-Hybridisierung. Beispielsweise kann ein Markierungsmittel an ein erfindungsgemäßes Aptamer gebunden werden, wie in WO2005113817 beschreiben. WO2005113817beschreibt ein Aptamer mit einem angefügten Oligonukleotidschwanz (oligonucleotide tail) und einem Linker-Oligonukleotid, der ein Label trägt, wobei die Sequenz des Linker-Oligonukleotid komplementär zu der Sequenz des Oligonukleotidschwanzes des Aptamers ist, sodass das Linker-Oligonukleotid an den Oligonukleotidschwanz des Aptamers hybridisiert und das Aptamer mit dem Label markiert wird. Eine weitere Möglichkeit ist die Bindung von Biotin an das Aptamer und die Bindung von Markierungsmittel an Steptavidin. Durch eine Biotin-Streptavidin Bindung wird das Markierungsmittel an das Aptamer gebunden.

Alle Markierungsmittel sind grundsätzlich einsetzbar, die sich an DNA oder RNA binden lassen. Konkrete Beispiele für Markierungssubstanzen wurden weiter oben bei der Erläuterung von erfindungsgemäßen Verfahren angegeben, bei denen markierte Aptamere eingesetzt werden können. Beispielsweise ist die Markierung ein Farbstoff. In einer speziellen Variante ist das Markierungsmittel eine Substanz, die eine Bilderzeugung durch das markierte Aptamer in einem bildgebenden Verfahren ermöglicht. Das Bild kann durch Strahlung erzeugt sein, die ein für das menschliche Auge direkt sichtbares Bild erzeugt, oder durch Strahlung, die nicht direkt sichtbar ist, beispielsweise Röntgenstrahlung oder radioaktive Strahlung, die anderweitig sichtbar gemacht wird, beispielsweise auf einem Film. Markierungsmittel können beispielsweise Lumineszenz-, Phosphoreszenz, Fluoreszenz-, radioaktive, oder UV/VIS- Markierungssubstanzen sein. Die erfindungsgemäße Sonde ist besonders für die in dieser Beschreibung erläuterten Nachweis-, Anreicherungs-, Abtrennungs- und/oder Isolierungsverfahren einsetzbar, insbesondere in Assays zur Detektion von Verbindungen aus der Gruppe der Aminoglykoside. Assays können z.B. direkte und indirekte Sandwich-Assays sein.

In einem weiteren Aspekt betrifft die Erfindung einen Biosensor, der ein erfindungsgemäßes Aptamer aufweist.

Ein solcher Biosensor weist gemäß der vorliegenden Erfindung vorzugsweise die folgenden Elemente auf:
- einen Rezeptor, aufweisend oder bestehend aus einem erfindungsgemäßen Aptamer, und
- einen Transducer, der das Bindungsereignis zwischen Aptamer und Target in ein elektrisch quantifizierbares Signal wandelt.

Außerdem können weitere Elemente, wie beispielsweise eine Signalverarbeitungseinrichtung, eine Ausgabeelektronik, eine Anzeigevorrichtung, eine Datenverarbeitungseinrichtung, ein Datenspeicher sowie Schnittstellen zu anderen Geräten vorhanden sein.

Der biologische Rezeptor des Aptamer-Biosensors weist das erfindungsgemäße Aptamer vorzugsweise in immobilisierter Form auf oder besteht nur aus dem Aptamer in immobilisierter Form. Die Funktion des Rezeptors ist die auf einem biochemischen Mechanismus beruhende Erkennung des Analyten, hier die Bindung des erfindungsgemäßen Aptamers an sein Target. Aus einer Probe, die man mit dem Biosensor in Kontakt bringt, beispielsweise einem komplexen Gemisch, das Target enthält, kann das Target über die spezifische Anbindung des Aptamers identifiziert werden.

Die Spezifität des Biosensors wird durch den Rezeptor vorgegeben, während die Sensitivität des Sensors vorwiegend durch den verwendeten Transducer beeinflusst wird. Die am Rezeptor stattfindende Aptamer-Target Bindung wird durch den Transducer in ein elektronisch verwertbares Signal umgesetzt. Der Transducer wandelt das Signal aus der selektiven Erkennungsreaktion des Rezeptors (Bindung Target an Aptamer), das der Konzentration des Targets in der Probe proportional ist, in ein elektrisch quantifizierbares Messsignal um. Die Signalbildung erfolgt auf Grund der molekularen Interaktion zwischen dem Target und dem Aptamer.

Mit einem erfindungsgemäßen Biosensor können vorzugsweise qualitative, quantitative und/oder halb-quantitative analytische Informationen gewonnen werden. Er ist insbesondere zum Einsatz in der Lebensmittel-, Wasser- und Umweltanalytik, der Wasserbehandlung und der Wasseraufbereitung, insbesondere von Trinkwasser und Abwasser, in der Diagnostik sowie zur Verwendung in Krankenhäusern und Pflegeeinrichtungen geeignet.

Die Kopplung zwischen Rezeptor und Transducer erfolgt vorzugsweise durch die Immobilisierung des Aptamers auf der Transduceroberfläche, beispielsweise über eine Straptavidin-Biotin-Bindung, wobei vorzugsweise das Aptamer mit Biotin verbunden ist und die Oberfläche des Transducers immobilisiertes Streptavidin aufweist.

Die Bindung des Targets an das erfindungsgemäße Aptamer kann beispielsweise über optische, mikrogravimetrische, thermische oder akustische Transducer gemessen werden, vorzugsweise über optische oder mikrogravimetrische Transducer.

Die Messung in optischen Transducern kann auf Prinzipien der Photometrie beruhen, wobei beispielsweise Farb- oder Lumineszenz- Intensitätsänderungen erfasst werden. Zu den optischen Methoden zählen die Messung von Fluoreszenz, Phosphoreszenz, Biolumineszenz und Chemolumineszenz, Infrarotübergängen und die Lichtstreuung. Zu den optischen Methoden zählt auch die Messung von Schichtdickenänderungen wenn Target an Aptamer gebunden wird. Die Schichtdickenänderung kann z.B. mittels Oberflächenplasmonenresonanz (SPR) oder reflektometrischer Interferenzspektroskopie (RIfS) durchgeführt werden. Ferner kann die Interferenz an dünnen Schichten (reflektrometrische Interferenzspektroskopie) sowie die Änderung des evaneszenten Feldes gemessen werden.

Mikrogravimetrische Transducer sind zum Beispiel QCM-Sensoren (Quartz Crystal Microbalance), die eine Masseänderung detektieren, wenn Target an Aptamer gebunden wird.

Akustische Transducer nutzen die Frequenzänderungen eines piezoelektrischen Schwingquarzes, der Masseänderungen hochempfindlich nachweist, die auftreten wenn Target an Aptamer bindet. Der verwendete Quarzkristall wird in einem oszillierenden elektrischen Feld platziert und die Resonanzfrequenz des Kristalls gemessen. Eine Masseänderung auf der Oberfläche des Schwingquarzes, z.B. durch Reaktion des Analyten mit dem vorher auf der Kristalloberfläche immobilisierten Rezeptor, bewirkt eine Änderung der Resonanzfrequenz, welche quantifiziert werden kann.

Elektrochemische Transducer können z.B. die Änderung der Konzentration redoxaktiver Marker an der Elektrodenoberfläche messen, oder die Änderung der Konzentration redoxaktiver Substrate oder Produkte, die z.B. in der enzymatischen Reaktion eines Enzym-Markers verbraucht werden oder entstehen.

Thermische Transducer messen die Wärmetönung der Aptamer-Target-Bindungsreaktion.

Ein beispielhafter Biosensor ist im beigefügten Ausführungsbeispiel erläutert. Anleitung zur Konstruktion weiterer Biosensoren findet der Fachmann in Cho, E. J., Lee, J.-W., Ellington, A. D. (2009): Applications of Aptamers as Sensors, Annual Review of Analytical Chemistry 2: 241-264; Mok, W and Li, Y. (2008): Recent Progress in Nucleic Acid Aptamer-Based Biosensors and Bioassays, Sensors 8: 7050-7084; Song, S., Wang, L., Li, J., Fan, C., Zhao, J. (2008): Aptamer-based biosensors, TrAC Trends in Analytical Chemistry 27: 108-117.

Die Erfindung betrifft in einem weiteren Aspekt eine feste Phase, auch bezeichnet als fester Träger, insbesondere zur Verwendung beim Nachweis, Anreicherung, Abtrennung und/oder Isolierung einer oder mehrerer Verbindungen aus der Gruppe der Aminoglykoside, insbesondere einer oder mehrerer Verbindungen aus der Gruppe der Kanamycine oder Paromomycin, an dem ein erfindungsgemäßes Aptamer immobilisiert ist.

Feste Phasen sind in allen möglichen geometrischen Formen denkbar. Beispiele für feste Träger sind Platten, Streifen, Membranen, Mikrotiterplatten, Filme, Gele, Mikropartikel, Nanopartikel oder Perlen. Besonders geeignete Materialien, aus denen eine feste Phase hergestellt werden kann, sind anorganische Polymere, organische Polymere, Gläser, organische und anorganische Kristalle, Keramiken, Metalle, insbesondere Edelmetalle, und Halbleiter. Ein besonders geeignetes organisches Polymer ist ein Polymer auf Basis von Polystyrol. Des Weiteren sind Biopolymere, vorzugsweise Cellulose, Dextran, Agar, Agarose und Sephadex, die funktionalisiert sein können, insbesondere als Nitrocellulose oder Cyanbromid-Sephadex, als Polymere einsetzbar. Aptamere können an magnetische Kügelchen (Magnetic Beads) gebunden sein, die z.B. Carboxy-terminierte oder Epoxyaktivierte Seitenketten tragen. Des Weiteren ist eine Kopplung von Magnetic Beads an die Ribose oder Desoxyribose der Aptamere, wie z.B. über eine Hydrazon-Bindung, möglich. Die Beispiele für Polymere sind nicht limitierend und andere Moleküle denkbar. Bevorzugte Kombinationen von geometrischer Form und Material sind Gele aus Biopolymeren, insbesondere Agarose, und Gelmatrices aus Dextran und Sephadex, die - beispielsweise in Säulen verpackt - Hohlräume definierter Porengröße ausbilden. In einer speziellen Ausführungsform ist der feste Träger mit immobilisiertem Aptamer eine stationäre Phase für die Affinitätschromatographie, wobei der Träger vorzugsweise die Form von Kugeln oder anderweitig geformten Partikeln hat.

In einer speziellen Ausführungsform ist die feste Phase ein Teststreifen, der ein oder mehrere verschiedene erfindungsgemäße Aptamere enthält. Der Teststreifen ist insbesondere einsetzbar für qualitative, halb-quantitative und quantitative Assays, die mit visuellen Nachweisverfahren arbeiten, insbesondere für Lateralfluss-Assays.

Ein Lateralfluss-Assay arbeitet wie folgt: Eine flüssige Probe, die mutmaßlich ein Target für das erfindungsgemäße Aptamer enthält, wird an einer Stelle auf den Streifen aufgebracht bzw. der Streifen an einer Stelle mit der Probe benetzt. Diese Stelle ist die sogenannte Probeauftragszone des Streifens. Der Streifen enthält ein Matrixmaterial, durch welches das flüssige Testmedium und das darin suspendierte oder gelöste Target durch Kapillarwirkung aus einer Probeauftragszone in eine Nachweiszone strömen kann, wo ein nachweisbares Signal oder die Abwesenheit eines solchen Signals auf das Vorhandensein des Targets hinweist.

Der Streifen, der für Lateralfluss-Assays einsetzbar ist, enthält ein oder mehrere erfindungsgemäße Aptamere, beispielsweise in der Probeauftragszone oder zumindest noch örtlich vor der Nachweiszone. Wenn Target in der Testprobe vorhanden ist bildet es mit dem im Streifen vorhandenen Aptamer einen Komplex, der zur Nachweiszone strömt und dort detektiert wird. Anders ausgedrückt strömt das Target innerhalb des Streifens zu dem Aptamer, bildet mit diesem einen Komplex, welcher anschließend zur Nachweiszone strömt.

Die Bindungsreaktion kann auch direkt auf der Auftragsstelle stattfinden, wenn z.B. der Teststreifen das Aptamer enthält und die Aptamer-Target-Bindung direkt mit einem entsprechenden Marker sichtbar gemacht wird.

Vorzugsweise wird ein markiertes Aptamer eingesetzt, wobei jede bereits weiter oben beschriebene Markierung einsetzbar ist. Vorzugsweise wird eine Markierung eingesetzt, die in der Nachweiszone des Teststreifens zu einem visuell detektierbaren Signal führt. Grundsätzlich kann das Vorhandensein oder die Abwesenheit von Target in der Probe durch Nachweis oder fehlenden Nachweis eines markierten Aptamers in der Nachweiszone ermittelt werden.

In einer Ausführungsform eines Teststreifens wird ein Enzym-markiertes Aptamer eingesetzt. In der Probe vorhandenes Target bildet mit dem Enzym-markierten Aptamer einen Komplex, der entlang des Streifens zu einer Nachweiszone strömt, die ein Substrat für den Enzymmarker enthält, der in Anwesenheit des Enzymmarkers eine farbige Reaktion zu erzeugen vermag. Der Streifen enthält vorzugsweise eine weitere Zone, in der Target immobilisiert ist, so dass markiertes Aptamer, das sich aufgrund der Abwesenheit von ausreichendem Target in der Probe nicht mit dem Target vereinigt, erfasst wird und dadurch gehindert wird, die Nachweiszone zu erreichen.

In einer weiteren Ausführungsform funktioniert der Teststreifen nach dem Prinzip eines immunologischen Lateralfluss-Assays, beispielsweise eines Schwangerschaftsteststreifens, wobei ein dort verwendetes markiertes Immunglobulin durch ein erfindungsgemäßes, vorzugsweise markiertes, Aptamer ersetzt ist.

Eine spezielle Variante funktioniert nach folgendem Prinzip: Ein Teststreifen wird mit Probe benetzt und in der Probe vorhandenes Target bindet an ein Farbstoff-markiertes Aptamer. Der Target-Aptamer-Farbstoff-Komplex wandert zur Nachweiszone, in der ein Antikörper fixiert ist, welcher ebenfalls an das Target bindet. Der immobilisierte Antikörper bindet den wandernden Target-Aptamer-Farbstoff-Komplex in der Nachweiszone und diese wird angefärbt. Überschüssiges Farbstoff-markiertes Aptamer wandert weiter zu einer Kontrollzone, in der eine Substanz fixiert ist, beispielsweise eine Verbindung aus der Gruppe der Aminoglykoside, die das Farbstoff-markierte Aptamer bindet, und dadurch angefärbt wird.

Der Teststreifen ist aus jedem Material herstellbar, das mit einer Probe benetzbar ist und in das ein erfindungsgemäßes Aptamer eingebracht werden kann. Insbesondere bevorzugt sind Materialien durch die eine Probe und ein darin enthaltenes Target durch Kapillarwirkung strömen können. Beispiele sind Nitrozellulose, Nitrozellulosemischungen mit Polyester oder Zellulose, unbeschichtetes Papier, poröses Papier, Viskosefilament, Glasfaser, Acrylnitrilcopolymer oder Nylon, sowie alle weiteren Materialien, die bei Lateralfluss-Assays üblich sind.

Der Teststreifen kann bereits als solcher verwendet werden, um das Vorhandensein von Target in einer Probe festzustellen. Zur Anwendung kann der Streifen in eine Probe eingetaucht werden, im Falle eines Lateralfluss-Assays beispielsweise mit nur einem Ende, das dann als Probeauftragszone dient.

Gegenstand der Erfindung ist auch eine Lateralfluss-Assay-Vorrichtung, die den oben beschriebenen Teststreifen aufweist. Die Vorrichtung kann neben dem Teststreifen beispielsweise ein Gehäuse aufweisen, in das der Teststreifen eingebettet ist und das eine, vorzugsweise verschließbare, Öffnung zur Probenauftragszone des Teststreifens aufweist, sowie Aussparungen zur Beobachtung einer Nachweis und ggf. Kontrollzone. Ein solcher Aufbau ist aus dem Gebiet der Schwangerschaftstests bekannt und entsprechend sind bekannte Aufbauten von Lateralfluss-Assay-Vorrichtung auch in der vorliegenden Erfindung verwendbar.

In einer anderen speziellen Ausführungsform bilden der feste Träger und das Aptamer ein Microarray oder einen sogenannten "DNA oder RNA-Chip", der einkanalig oder mehrkanalig sein kann. Im Microarray können auf mehreren Array-förmig angeordneten Messpunkten ein oder mehrere Aptamere sowie Referenzmaterialien zur Grundsignalkompensation bzw. Funktionstestung angeordnet sein. In einem Mehrkanal-Chip erfolgt diese Anordnung in den einzelnen Kanälen. Dadurch ist die parallele Mehrfachmessung einer Probe, oder bei verschiedenen Aptameren, die parallele Messung unterschiedlicher Analyte in einer Probe möglich.

Die Immobilisierung von Aptamer an fester Phase kann auf verschiedene Art und Weise erfolgen und auf jegliche Art und Weise, die dem Fachmann zur Immobilisierung von DNA oder RNA an Feststoffen bekannt ist. Bekannte Möglichkeiten wurden bereits zuvor in dieser Beschreibung genannt. Die Immobilisierung von Aptameren auf Nanopartikeln ist z.B. beschrieben in WO2005/13817. Eine feste Phase aus Papier oder einem porösen Material kann mit dem Aptamer in flüssiger Phase benetzt und die flüssige Phase anschließend verflüchtigt werden, so dass das Aptamer in dem Papier bzw. dem porösen Material zurückbleibt.

In einem weiteren Aspekt betrifft die vorliegende Erfindung auch ein Kit umfassend ein erfindungsgemäßes Aptamer.

Das Kit umfasst vorzugsweise ein erfindungsgemäßes Aptamer, insbesondere ein markiertes erfindungsgemäßes Aptamer bzw. eine erfindungsgemäße Aptamersonde, sowie weitere Komponenten für die mit dem Kit beabsichtigte Reaktion oder das mit dem durchzuführende Verfahren, beispielsweise Komponenten für ein beabsichtigtes Nachweis Anreicherungs-, Abtrennungs- und/oder Isolierungsverfahren. Beispiele sind Pufferlösungen, Substrate für eine Farbreaktion, Farbstoffe oder enzymatische Substrate. Das Aptamer und/oder weitere Komponenten können an einer festen Phase immobilisiert sein. Beispielhafte Formen und Materialien für feste Phasen wurden bereits an anderer Stelle in dieser Beschreibung genannt. Die feste Phase kann beispielsweise in Form eines (Micro)-Arrays oder einer Mikrotiterplatte-bereitgestellt werden. Die feste Phase kann im konkreten Fall eine immobilisierte Fängersubstanz für das Target aufweisen, insbesondere einen immobilisierten Antikörper, der zur Anbindung von Target dient. Die immobilisierte Fängersubstanz ist vorzugsweise in Form eines (Micro)-Arrays, in einer Mikrotiterplatte oder in Chips angeordnet.

Das Kit dient vorzugsweise zur Durchführung eines erfindungsgemäßen Nachweis-, Anreicherungs-, Abtrennungs- und/oder Isolierungsverfahrens, oder zur Durchführung anderweitiger Assays unter Zuhilfenahme eines erfindungsgemäßen Aptamers. Insofern wird auf die vorangehende Offenbarung Bezug genommen.

In dem Kit kann das Aptamer in verschiedensten Formen bereitgestellt werden, beispielsweise gefriergetrocknet oder in einem flüssigen Medium.

Die Erfindung betrifft auch ein Messgerät zum Nachweis einer oder mehrerer Verbindungen aus der Gruppe der Aminoglykoside, insbesondere einer oder mehrerer Verbindungen aus der Gruppe der Kanamycine oder Paromomycin. Das Messgerät weist ein oder mehrere verschiedene der zuvor beschriebenen Aptamere, eine Aptamersonde wie zuvor beschrieben oder einen Biosensor wie zuvor beschrieben auf. Darüber hinaus kann das Messgerät unter anderem eine Probeentnahmeeinrichtung, eine Signalverarbeitungseinrichtung, eine Anzeigevorrichtung zum Ablesen von Messergebnissen oder Messwerten, eine Datenverarbeitungseinrichtung, einen Datenspeichereinrichtung und Schnittstellen zur Verbindung mit externen Geräten oder Speichermedien aufweisen.

Mit dem Messgerät können je nach Ausgestaltung qualitative, quantitative und/oder halb-quantitative analytische Informationen über das zu messende Target gewonnen werden. Mit dem Messgerät können die zuvor erläuterten Nachweisverfahren durchgeführt werden. Es ist insbesondere zum Einsatz in der Lebensmittel-, Wasser- und Umweltanalytik, der Wasserbehandlung und der Wasseraufbereitung, insbesondere von Trinkwasser und Abwasser, in der Diagnostik sowie zur Verwendung in Krankenhäusern und Pflegeeinrichtungen geeignet.

Das Messgerät ist insbesondere ein transportables Messgerät, das vor Ort eingesetzt werden kann, beispielsweise ein leichtes Messgerät im Taschenformat.

Die Probeentnahmeeinrichtung des Messgeräts kann auf verschiedene Art und Weise aufgebaut sein. In einer Variante ist die Probeentnahmeeinrichtung eine Kapillare oder ein poröser Streifen, im dem Flüssigkeiten aufgesaugt werden können. Eine solche Probeentnahmevorrichtung wird zur Probeentnahme üblicherweise in eine flüssige Probe eingetaucht. Durch Kapillarkraft wird die Probe zu dem gewünschten Ort im Messgerät transportiert, an dem sich das Aptamer befindet und wo die Nachweisreaktion stattfinden kann. Beispielsweise wird die Probe zu einem das Aptamer aufweisenden Biosensor transportiert, welcher seinerseits ein Messsignal erzeugt.

In einer anderen Variante weist die Probeentnahmeeinrichtung einen Schlauch oder ein Röhrchen sowie eine Pumpe auf. Mit der Pumpe wird eine flüssige Probe angesaugt und die Probe zu dem gewünschten Ort im Messgerät transportiert, an dem sich das Aptamer befindet und wo die Nachweisreaktion stattfinden kann. Beispielsweise wird die Probe zu einem das Aptamer ausweisenden Biosensor transportiert, welcher seinerseits ein Messsignal erzeugt.

Die Erfindung betrifft in einem Aspekt auch die Verwendung einer zuvor beschriebenen Sonde, einer zuvor beschriebenen festen Phase, eines zuvor beschriebenen Biosensors, eines zuvor beschriebenen Teststreifens, einer zuvor beschriebenen Lateralfluss-Assay-Vorrichtung, eines zuvor beschriebenen Kits, oder eines zuvor beschriebenen Messgeräts zum Nachweis einer oder mehrerer Verbindungen aus der Gruppe der Aminoglykoside, oder zur Anreicherung, Abtrennung und/oder Isolierung einer oder mehrerer Verbindungen aus der Gruppe der Aminoglykoside. Hierbei wird auf alle zuvor beschriebenen Verfahrensvarianten Bezug genommen.

Im Folgenden wird die Erfindung anhand von nicht limitierenden Beispielen für konkrete Ausführungsformen näher erläutert. In den Beispielexperimenten werden Standardreagenzien und Puffer verwendet, die frei von Kontaminationen sind.

### BEISPIEL 1: Präparation der Magnetic Beads.

Dynabeads® M-280 Streptavidin Magnetic Beads (Dynal Biotech, Norwegen) wurden nach Herstellerangaben verwendet. Alle für die folgenden Schritte notwendigen 1,5 mL-Reaktionsgefäße wurden vor ihrer Verwendung gewaschen: 1 x mit Bindungspuffer mit Detergenz-Zusatz: (BB+(KC): 20 mmol L⁻¹ Tris-HCl pH 7.6, 100 mmol L⁻¹ NaCl, 2 mmol L⁻¹ MgCl₂, 5 mmol L⁻¹ KCl, 1 mmol L⁻¹ CaCl₂, 0,02 % Tween 20 sowie 2 x mit Bindungspuffer ohne Zusatz von Tween (BB(KC)). 4 × 10⁹ Magnetic Beads wurden 1 h bei 21 °C und leichtem Schütteln mit 600 pmol der Fängersequenzen in 700 µL Puffer inkubiert. Danach folgten Waschschritte: 3 x in 10 mmol L⁻¹ Tris-HCl pH 7.5, 1 mmol L⁻¹ EDTA, 2 mol L⁻¹ NaCl und 3 x in BB(KC). Die Abtrennung der Beads von den Waschlösungen erfolgte jeweils unter Verwendung eines Magnet-Separationsstandes (Promega, Deutschland). Die mit den Fängersequenzen modifizierten Beads wurden dann in 4200 µL BB(KC) resuspendiert und bei 4 °C aufbewahrt.

### BEISPIEL 2: In-vitro Selektion (Capture-SELEX).

Die DNA-Aptamer-Selektion wurde unter Anwendung des Capture-SELEX-Prozesses durchgeführt. Eingefügte Fluoreszenz-Marker erlauben die Quantifizierung der DNA nach verschiedenen Schritten des Prozesses mittels Fluoreszenz-Messung. Die eine Bindung der ssDNA-Oligomeren aus der Ausgangsbibliothek bzw. ihrer Derivate an Magnetic Beads erfolgt über die Fängersequenz (Capture sequence). Die Fängersequenz ist 12 Basen lang und zu einem Bereich in der ssDNA der Ausgangsbibliothek komplementär. Mit Hilfe der Fängersequenz findet eine reversible Bindung zwischen Fänger und ssDNA statt. An ihrem 5'-Ende trägt die Fängersequenz über einen Hexaethylenglycol(HEGL)-Rest gebundenes Biotin, welches für die Ankopplung der Fängersequenz an die Streptavidin-modifizierten Magnetic Beads verantwortlich ist.
Die Targetsubstanzen (Kanamycin A-disulfat-dihydrat, Sulfacarbamid, Sulfamethoxazol, Sotalol HCl), für welche DNA-Aptamere selektiert werden sollten, lagen frei in Lösung in einer Mischung mit einer Konzentration von je 1 mM vor und standen für eine Bindung mit der ssDNA zur Verfügung. Targetspezifische Oligomere sollten sich durch Ausbildung einer definierten zwei- oder dreidimensionalen Struktur von der Fängersequenz abkoppeln und zusammen mit dem Target in Lösung zu finden sein.

Ausgangspunkt des Selektionsprozesses war eine ssDNA-Bibliothek mit der grundsätzlichen Struktur: 5'-ATACCAGCTTATTCAATT-N₁₀-TGAGGCTCGATC-N₄₀-AGATAGTAAGTGCAATCT-3', wobei N für eine beliebige der vier Basen A, T, C oder G steht. Folgende modifizierte Primer wurden in der Amplifizierung der Oligomere mittels PCR eingesetzt: Sense-Primer AP60 mit der Sequenz: 5' Fluorescein-ATACCAGCTTATTCAATT-3' (siehe SEQ ID NO: 59) und Antisense-Primer TER-AP20 mit der Sequenz: 5' dA₂₀-HEGL-AGATTGCACTTACTATCT-3' (dA20 = 20 Nukleotide mit der Base Adenin, HEGL = Hexaethylenglycolspacer, Primersequenz SEQ ID 62) (Alle chemisch synthetisiert von Microsynth, Schweiz.). Die Bibliothek wurde mittels PAGE gereinigt. Primer wurden mit HPLC gereinigt. Die Fängersequenz hatte folgende Struktur: 5' Bio-GTC-HEGL-GATCGAGCCTCA-3' (SEQ ID NO: 63) und wurde von IBA (Deutschland) hergestellt sowie HPLC gereinigt.

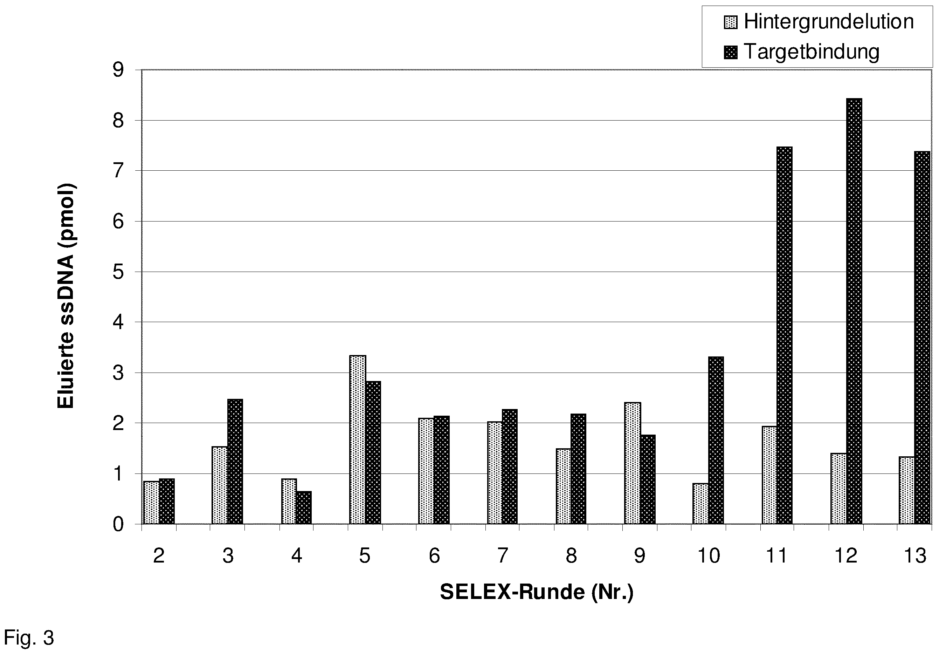

Es wurden aufeinanderfolgende Runden des SELEX-Prozesses durchgeführt, die sich aus den folgenden Schritten zusammensetzten: (i) Bindung der ssDNA-Bibliothek bzw. der in der vorhergehenden Runde selektierten ssDNA an die mit der Fängersequenz modifizierten Beads, (ii) Abtrennen der ungebundenen ssDNA, (iii) Ablösen von nur schwach gebundenen Oligomeren in einem Temperaturschritt (iv) Hintergrund-Elution in Puffer bei gleichen Bedingungen wie bei der nachfolgenden Target-Elution jedoch ohne Target (v) Target-Elution in Anwesenheit der Mischung der Targetsubstanzen, (vi) Vervielfältigung der eluierten ssDNA mittels PCR, (vii) Trennung des Doppelstranges aus der PCR und Gewinnung des relevanten Einzelstranges. In jeder Runde wurden 10⁸ mit der Fängersequenz modifizierten Magnetic Beads eingesetzt (Ausnahme: 1. Runde, 10⁹ Beads). Nach der 12. Runde wurden die selektierten Oligonukleotide auf ihre Bindungsspezifität für die vier in der Mischung enthaltenen Targetsubstanzen getestet. Dabei wurde eine eindeutige Anreicherung von Kanamycin A-bindender ssDNA gefunden. Die Fig. 3 zeigt die Anreicherung von Aptameren über 13 Runden Capture-SELEX für die Target-Mischung, bestehend aus Kanamycin A Disulfatsalz Dihydrat, Sulfcarbamid, Sulfamethoxazol und Sotalol-Hydrochlorid, jeweils 1 mM in Selektionspuffer. Die Oligonukleotide wurden mit Fluorescein zur Detektion gelabelt, wie oben beschrieben. Die Menge eluierter ssDNA in dem Hintergrund-Elutionsschritt ist in hellen Balken gezeigt, die Menge eluierter einzelsträngiger DNA (ssDNA) in dem Target-Bindungsschritt ist als dunkle Balken gezeigt. Nach der Runde 10 wird ein Anstieg eluierter ssDNA in dem Target-Bindungsschritt festgestellt. In der Runde 13 sinkt die Menge wieder, was das Ende des SELEX-Prozesses anzeigt. Da die Start-Oligonukleotid-Bibliothek anfänglich nicht mit Fluorescein gelabelt war, sind für die Runde 1 keine Balken angegeben.

### BEISPIEL 3: Klonierung and Sequenzierung.

In der 13. und letzten SELEX-Runde wurde die selektierte ssDNA in der PCR mit unmodifizierten Primern amplifiziert, um sie danach in den Vektor pCR2.1-TOPO zu klonieren. *E. coli* TOP10 Zellen (TOPO TA Cloning Kit; Invitrogen, U.K.) wurden durch Aufnahme dieses Vektor-Konstruktes transformiert. Unter Verwendung des QIAprep 96 Turbo Miniprep Kit (Qiagen, Deutschland) wurde die Plasmid-DNA aus 96 der erhaltenen Klone präpariert und zur Sequenzierung gegeben (Microsynth, Schweiz). Sequenz-Analysen und Alignments wurden unter Verwendung der Webseite ClustalW2 (http://www.ebi.ac.uk/Tools/msa/clustalw2/) durchgeführt. Die Analyse der Sekundärstruktur einiger Aptamere erfolgte mittels des im Internet verfügbaren Faltungsprogramms mfold, das auf einem Energie-Minimierungs-Algorithmus beruht.

Eine Übersicht der aus der Klonierung erhaltenen Aptamersequenzen ist in der Tabelle 1 angegeben. Die Primersequenz (Sense) am 5'-Ende ist ATACCAGCTTATTCAATT (SEQ ID NO: 59) und die Primerbindungsregion für den Antisense-Primer am 3'-Ende ist AGATAGTAAGTGCAATCT (SEQ ID NO: 60, komplementär zur Antisense-Primersequenz). Der komplementäre Bereich für die Fängersequenz (Docking-Sequenz) für den Capture-Selex Prozess ist TGAGGCTCGATC (SEQ ID NO: 61). Diese Sequenzen, und davon abgewandelte Sequenzen, sind jeweils in Fettschrift dargestellt. Die Sequenzen mit den SEQ ID Nos. 59-61 sind Bestandteil der Aptamere, vorbehaltlich verkürzter Sequenzen, wie in der vorangehenden allgemeinen Beschreibung definiert.

Einige Aptamere lassen sich aufgrund ihrer Sequenzen in Gruppen einteilen. Innerhalb einer Gruppe im Vergleich zum Stellvertreter einer Gruppe abweichende Nukleotide sind unterstrichen gekennzeichnet. Die in einer Gruppe zusammengefassten Sequenzen sind bis auf Punktmutationen und Deletionen identisch bzw. vom Stellvertreter abzuleiten. Die Aptamernummer des Stellvertreters einer Gruppe ist unterstrichen gekennzeichnet (siehe z.B. Aptamer Nr. 3-7 für Gruppe 1). Unterstrichene Nukleotide oder Deletionen (-) innerhalb des komplementären Bereichs der Fängersequenz bedeuten eine Abwandlung gegenüber dem ursprünglichen komplementären Bereich der Fängersequenz TGAGGCTCGATC (SEQ ID NO: 61).

Die Spalte "Anzahl" gibt die Anzahl Klone mit identischer Aptamersequenz an. Wenn mehrere Klone mit identischer Sequenz gefunden wurden, ist die Aptamerbezeichnung in Fettschrift markiert (siehe z.B. Aptamer Nr. 3-7).

Die Sequenz mit der SEQ ID NO: 45 fasst die Sequenzen der Gruppe 1 (erfindungsgemäß) zusammen und zeigt variable Nukleotide unterstrichen.
Die Sequenz mit der SEQ ID NO: 46 fasst die Sequenzen der Gruppe 2 (nicht erfindungsgemäß) zusammen und zeigt variable Nukleotide unterstrichen.
Die Sequenz mit der SEQ ID NO: 47 fasst die Sequenzen der Gruppe 3 (nicht erfindungsgemäß) zusammen und zeigt variable Nukleotide unterstrichen.
Die Sequenz mit der SEQ ID NO: 48 fasst die Sequenzen der Gruppe 4 (erfindungsgemäß) zusammen und zeigt variable Nukleotide unterstrichen.
Die Sequenz mit der SEQ ID NO: 49 fasst die Sequenzen der Gruppe 5 (nicht erfindungsgemäß)zusammen und zeigt variable Nukleotide unterstrichen.
Die Sequenz mit der SEQ ID NO: 50 fasst die Sequenzen der Gruppe 6 (erfindungsgemäß) zusammen und zeigt variable Nukleotide unterstrichen.
Die Sequenz mit der SEQ ID NO: 51 fasst die Sequenzen der Gruppe 7 (erfindungsgemäß) zusammen und zeigt variable Nukleotide unterstrichen.
Die Sequenz mit der SEQ ID NO: 52 fasst die Sequenzen der Gruppe 8 (erfindungsgemäß) zusammen und zeigt variable Nukleotide unterstrichen.
Die Sequenz mit der SEQ ID NO: 53 fasst die Sequenzen der Gruppe 9 (erfindungsgemäß) zusammen und zeigt variable Nukleotide unterstrichen.
Die Sequenz mit der SEQ ID NO: 54 fasst die Sequenzen der Gruppe 10 (erfindungsgemäß) zusammen und zeigt variable Nukleotide unterstrichen.

In der SEQ ID NO:45 bedeutet X₁ entweder G oder L, und X₂ bedeutet entweder A oder L wobei L für kein Nukleotid (Deletion) steht.

In der SEQ ID NO:52 bedeutet X₃ entweder C oder L, wobei L für kein Nukleotid (Deletion) steht.

Die Tabelle 2 zeigt eine Übersicht der Nukleotidsymbole, die für eines oder mehrere der Nukleotide A, G, C oder T stehen.

**Tabelle 1**

| **Aptamer-Nr.** | **Aptamersequenz (5' → 3')** | **SEQ ID NO** | **Anzahl** |
|---|---|---|---|
| **Gruppe 1** | | | |
| **3-7** | **ATACCAGCTTATTCAATT**GGTGGTAGGA**TG**-**GGCTCGATC**GAGGTCTTCAGTCGGCTCGGTTTAGTTTATGTTCTCCTGT**AGATAGTAAGTGCAATCT** | 1 | 4 |
| 3-8 | **ATACCAGCTTATTCAATT**GGTGGTAGGA**T**-**AGGCTCGATC**GAGGTCTTCAGTCGGCTCGGTTTAGTTTATGTTCTCCTGT**AGATAGTAAGTGCAATCT** | 2 | 1 |
| 3-14 | **ATACCAGCTTATTCAATT**GGTGGTAGGA**TG-GGCTCGATC**GAGGTCTTCAGTCGGCTCGGTCTAGTTTATGTTCTCCTGT**AGATAGTAAGTGCAATCT** | 3 | 5 |
| 4-29 | **ATACCAGCTTATTCAATT**GGTGGTAGGA**TG-GGCTCGATC**GAGGTCTTCAGTCGGCTCGGTCTAATTTATGTTCTCCTGT**AGATAGTAAGTGCAATCT** | 4 | 1 |
| 13-92 | **ATACCAGCTTATTCAATT**GGTGGTAGGA**TG**-**GGCTCGATC**GAGGTCTTCAGTTGGCTCGGTTTAGTTTATGTTCTCCTGT**AGATAGTAAGTGCAATCT** | 5 | 1 |
| 4-21 | **ATACCAGCTTATTCAATT**GGTGGTAGGA**TG**-**GGCTCGATC**GAGGTCTTTAGTCGGCTCGGTTTAGTTTATGTTCTCCTGT**AGATAGTAAGTGCAATCT** | 6 | 2 |
| 10-50 | **ATACCAGCTTATTCAATT**GGTGGTAGGA**TG**-**GGCTCGATC**GAGGTCTTTAGTCGGCTCGGTTTAGTTTATGTTCTCCTGC**AGATAGTAAGTGCAATCT** | 7 | 1 |
| 11-70 | **ATACCAGCTTATTCAATT**GGTGGTAGGA**TG**-**GGCTCGATC**GAGGTCTTTAGTCGGCTCGGTTTAGTTTATGTTCTCTTGT**AGATAGTAAGTGCAATCT** | 8 | 1 |
| 3-2 | **ATACCAGCTTATTCAATT**GGTGGTAGGA**TG**-**GGCTCGATC**GAGGTCTTCAGTCGGCTCGGTTTAGTTTATGTTCTTCTGT**AGATAGTAAGTGCAATCT** | 9 | 1 |
| 4-27 | **ATACCAGCTTATTCAATT**GTCAGTGGGGT**ATACCAGCTTATT**CAATTGGTGGTAGGA**TG-GGCTCGATC**GAGGTCTTCAGTCGGCTCGGTTTAGTTTATGTTCTCCTGT**AGATAGTAAGTGCAATCT** | 10 | 1 |
| | | | |
| | **ATACCAGCTTATTCAATT**GGTGGTAGGA**TX₁X₂GGCTCGATC**GAGGTCTTYAGTYGGCTCGGTYTARTTTATGTTCTYYTGY**AGATAGTAAGTGCAATCT** | 45 | |

| **Gruppe 2** | | | |
|---|---|---|---|
| **10-51** | **ATACCAGCTTATTCAATT**CGAGGGCTCG**TGAGGCTCGATT**TTGTGGTTTAAGAATTGTTAATGCGGTGTTAGTCTTGTGC**AGATAGTAAGTGCAATCT** | 11 | 12 |
| 10-52 | **ATACCAGCTTATTCAATT**CGAGGGCTCG**TGAGGCTCGATT**TTGTGGTTTAAGAATTGTTAATGCGATGTTAGTCTTGTGC**AGATAGTAAGTGCAATCT** | 12 | 1 |
| 11-67 | **ATACCAGCTTATTCAATT**CGAGGGCTCG**TGAGGCTCGATT**TTGTGGTTTAAGAATTGTTAATGCGGTGTTAGTCTTGTGT**AGATAGTAAGTGCAATCT** | 13 | 1 |
| 10-59 | **ATACCAGCTTATTCAATT**CGAGGGCTCG**CGAGGCTCGATT**TTGTGGTTTAAGAATTGTTAATGCGGTGTTAGTCTTGTCC**AGATAGTAAGTGCAATCT** | 14 | 1 |
| | **ATACCAGCTTATTCAATT**CGAGGGCTCG**YGAGGCTCGATT**TTGTGGTTTAAGAATTGTTAATGCGRTGTTAGTCTTGTSY**AGATAGTAAGTGCAATCT** | 46 | |
| | | | |

| **Gruppe 3** | | | |
|---|---|---|---|
| **13-83** | **ATACCAGCTTATTCAATT**GGGGAGATCT**TGAGGCTCGATC**GATAGTAGCTGTGACAGTCTACGTTTTATGGTATGCGGGT**AGATAGTAAGTGCAATCT** | 15 | 10 |
| 4-35 | **ATACCAGCTTATTCAATT**GGTGGGGT**ATACCAGCTTATTCAATT**GGGGAGATCT**TGAGGCTCGATC**GATAGTAGCTGTGACAGTCTACGTTTTATGGTATGCGGGT**AGATAGTAAGTGCAATCT** | 16 | 1 |
| | **ATACCAGCTTATTCAATT**GGGGAGATCT**TGAGGCTCGATC**GATAGTAGCTGTGACAGTCTACGTTTTATGGTATGCGGGT**AGATAGTAAGTGCAATCT** | 47 | |
| | | | |

| **Gruppe 4** | | | |
|---|---|---|---|
| **4-30** | **ATACCAGCTTATTCAATT**GGGGCCGATA**TGAGGCTTGATC**TAGTGTTTTAATTGGATTATTTGGGACTACTCTTGTTGCC**AGATAGTAAGTGCAATCT** | 17 | 3 |
| 10-44 | **ATACCAGCTTATTCAATT**GGGGCCGATA**TGAGGCTTGATC**TAGTGTTTTAATTGGATTATTTGGGATTACTCTTGTTGCC**AGATAGTAAGTGCAATCT** | 18 | 2 |
| 11-79 | **ATACCAGCTTATTCAATT**GGGGCCGATA**TGAGGCTTGATC**TAGTGTTTTAATTGGATTATTTGGGATTACTTTTGTTGCC**AGATAGTAAGTGCAATCT** | 19 | 1 |
| | **ATACCAGCTTATTCAATT**GGGGCCGATA**TGAGGCTTGATC**TAGTGTTTTAATTGGATTATTTGGGAYTACTYTTGTTGCC**AGATAGTAAGTGCAATCT** | 48 | |

| **Aptamer-Nr.** | **Aptamersequenz (5' → 3')** | **SEQ ID NO** | **Anzahl** |
|---|---|---|---|
| **Gruppe 5** | | | |
| **10-46** | **ATACCAGCTTATTCAATT**CGCGAGAGAA**TGAGGCTCGATC**TTGAGGGTTGTATGAGTATCAACAGTAGTGTTTGGTTTGC**AGATAGTAAGTGCAATCT** | 20 | 3 |
| 13-97 | **ATACCAGCTTATTCAATT**CGCGAGAGAG**TGAGGCTCGATC**TTGAGGGTTGTATGAGTATCAACAGTAGTGTTTGGTTTGC**AGATAGTAAGTGCAATCT** | 21 | 1 |
| 10-49 | **ATACCAGCTTATTCAATT**CGCGAGAGAA**TGAGGCTCGATC**TTGAGGGTTGTATGAGTATCAACAGTAGTGTTTGGTTTGT**AGATAGTAAGTGCAATCT** | 22 | 1 |
| | **ATACCAGCTTATTCAATT**CGCGAGAGAR**TGAGGCTCGATC**TTGAGGGTTGTATGAGTATCAACAGTAGTGTTTGGTTTGY**AGATAGTAAGTGCAATCT** | 49 | |
| | | | |

| **Gruppe 6** | | | |
|---|---|---|---|
| 13-82 | **ATACCAGCTTATTCAATT**CAGGGCGGTA**TGAGGCTCGATC**AAGGTCGGAGCGAGAATTTTTTCGCGGAGTCGGCTGGATC**AGATAGTAAGTGCAATCT** | 23 | 1 |
| 13-99 | **ATACCAGCTTATTCAATT**CAGGGCGGTA**TGAGGCTCGATT**AAGGTCGGAGCGAGAATTTTCTCGTGGAGTCGGCTGGATC**AGATAGTAAGTGCAATCT** | 24 | 1 |
| 13-90 | **ATACCAGCTTATTCAATT**CAGGGTGGTA**TGAGGCTCGATC**AAGGTCGGAGCGAGAACTTTTTCGCGGAGTCGGCTGGATC**AGATAGTAAGTGCAATCT** | 25 | 1 |
| 4-26 | **ATACCAGCTTATTCAATT**CAGGGCGGTA**TGAGGCTCGATC**AAGGTCAGAGTGAGAACTTTCTCGCGGAGTCGGCTGGATC**AGATAGTAAGTGCAATCT** | 26 | 1 |
| | **ATACCAGCTTATTCAATT**CAGGGYGGTA**TGAGGCTCGATY**AAGGTCRGAGYGAGAAYTTTYTCGYGGAGTCGGCTGGATC**AGATAGTAAGTGCAATCT** | 50 | |
| | | | |

| **Gruppe 7** | | | |
|---|---|---|---|
| **3-18** | **ATACCAGCTTATTCAATT**AGCATGGTGC**TGAGGCTTGATC**TGGAGGTCGTGAACGAAAATTGGTCGTTTAGTTGGCTCGT**AGATAGTAAGTGCAATCT** | 27 | 2 |
| 13-85 | **ATACCAGCTTATTCAATT**AGGCTAGGGT**TGAGGCTTGATC**TGGAGGTCGTGAACGAAAATTGGTCGTTTAGTTGGCTCGT**AGATAGTAAGTGCAATCT** | 28 | 1 |
| | **ATACCAGCTTATTCAATT**AGSMTRGKGY**TGAGGCTTGATC**TGGAGGTCGTGAACGAAAATTGGTCGTTTAGTTGGCTCGT**AGATAGTAAGTGCAATCT** | 51 | |

| **Gruppe 8** | | | |
|---|---|---|---|
| 3-4 | **ATACCAGCTTATTCAATT**ATGCGGGGA**TAAGGCTCGATT**TGTGGCTCTGATCCATGATCAGTCGTCTTACGTGCGGTCCAG**ATAGTAAGTGCAATCT** | 29 | 1 |
| 11-76 | **ATACCAGCTTATTCAATT**ATGCGGGGA**TGAGGCTTGATC**TGTGGCTCTGATCCATGATCAGTCGTCTTACGTGCGGTCCAG**ATAGTAAGTGCAATCT** | 30 | 1 |
| 4-23 | **ATACCAGCTTATTCAATT**GCTGCGGGGA**TGAGGCTCGGTC**TGTGGCTCTGATCCATGATCAGTCGTCTTACGTGCGGTCC**AGATAGTAAGTGCAATCT** | 31 | 1 |
| | **ATACCAGCTTATTCAATT**RX₃TGCGGGGA**TRAGGCTYGRTY**TGTGGCTCTGATCCATGATCAGTCGTCTTACGTGCGGTCC**AGATAGTAAGTGCAATCT** | 52 | |
| | | | |

| **Gruppe 9** | | | |
|---|---|---|---|
| 3-19 | **ATACCAGCTTATTCAATT**AGCCCGGTAT**TGAGG-TCGATC**TCTTAT**C**CTATGGCTTGTCCCCCATGGCTCGGTTATATCC**AGATAGTAAGTGCAATCT** | 32 | 1 |
| 10-56 | **ATACCAGCTTATTCAATT**AGCCTGGTAT**TGAGG-TCGATC**TCTTATCCTATGGCTTGTCCCCCATGGCTCGGTTATATCC**AGATAGTAAGTGCAATCT** | 33 | 1 |
| | **ATACCAGCTTATTCAATT**AGCCYGGTAT**TGAGG-TCGATC**TCTTATCCTATGGCTTGTCCCCCATGGCTCGGTTATATCC**AGATAGTAAGTGCAATCT** | 53 | |
| | | | |

| **Gruppe 10** | | | |
|---|---|---|---|
| 10-47 | **ATACCAGCTTATTCAATT**GCCAAGATGC**TGAGG**-----**TC**AATGTATTATGGAGGCTCGTTGTTGGCTCGGTCTTGCTGC**AGATAGTAAGTGCAATCT** | 34 | 1 |
| 11-77 | **ATACCAGCTTATTCAATT**GTCAAGATGC**TGAGG**-----**TC**AATGTATTATGGAGGCTCGTTGTTGGCTCGGTCTTGCTGC**AGATAGTAAGTGCAATCT** | 35 | 1 |
| | **ATACCAGCTTATTCAATT**GYCAAGATGC**TGAGGTC**AATGTATTATGGAGGCTCGTTGTTGGCTCGGTCTTGCTGC**AGATAGTAAGTGCAATCT** | 54 | |

| **Weitere Aptarmere** | | | |
|---|---|---|---|
| 3-1 | **ATACCAGCTTATTCAATT**GGTGGGAGTT**TGAGGTTCGATC**AAGGTCGAGATCGCTAGGGCGTCGTAGTTGGCTTGCTTGT**AGATAGTAAGTGCAATCT** | 36 | 1 |
| 4-25 | **ATACCAGCTTATTCAATT**GTCAGTGGGGT**ATACCAGCTTATTCAATT**GGGGTATAAT**TGAGGTTCGATC**AACGGGGGTCGGTCGCGGATAGTGTTAGATTTCTCTATGC**AGATAGTAAGTGCAATCT** | 37 | 1 |
| 4-28 | **ATACCAGCTTATTCAATT**ATGAGCGGGTG**TGAGGCTTGATC**AATAGAGGTCGATAGTTGGCTATATTGGCGTGTGTC**AGATAGTAAGTGCAATCT** | 38 | 1 |
| 4-31 | **ATACCAGCTTATTCAATT**GCGTAGATCA**TGAGGCTCGATC**TTGCGTGCGGTCCTAGTGGCTCAATTTCTTTAGTTGGTGC**AGATAGTAAGTGCAATCT** | 39 | 1 |
| 10-53 | **ATACCAGCTTATTCAATT**GAGGCGGGA-**TGAGGTTCGATC**TAGGTCAAAGTCGGCTAGATCATTGTGTGATGTTGTTTGC**AGATAGTAAGTGCAATCT** | 40 | 1 |
| 11-75 | **ATACCAGCTTATT**CAATTGTGAATCAGA--**AGGCTCGATC**TGTTGGCTTGTTAGAAGAGATGTGATACTATACTCGGTGT**AGATAGTAAGTGCAATCT** | 41 | 1 |
| 13-89 | **ATACCAGCTTATTCAATT**GGCGCAGTAT**TGAGGCTTGATC**AAAGGCTCGGGTCTAATACTTCCTCTTTTTGAGGGCCTGT**AGATAGTAAGTGCAATCT** | 42 | 1 |
| 13-91 | **ATACCAGCTTATTCAATT**ATGAGGTTAG**TGAGGCTCGATC**AAGGTCTTTAGTCGGCTTGATCACATTGGGTGTACGTTGT**AGATAGTAAGTGCAATCT** | 43 | 1 |
| 13-95 | **ATACCAGCTTATTCAATT**GCGTTAGGTA**TGAGGTTCGATC**GGCTCGTTATTTGTCGAGTATTCCAGTGTCAGGTTGGGGT**AGATAGTAAGTGCAATCT** | 44 | 1 |

**Tabelle 2**

| **Symbol** | **Bedeutung** |
|---|---|
| R | G oder A |
| Y | T/U oder C |
| M | A oder C |
| K | G oder T/U |
| S | G oder C |
| W | A oder T/U |
| B | G oder C oder T/U |
| D | A oder G oder T/U |
| H | A oder C oder T/U |
| V | A oder G oder C |

Die Tabelle 3 zeigt übereinstimmende Sequenzmotive beim Vergleich der Gruppen 1 und 6 der Tabelle 1. Die Stellvertreter der Gruppen 1 und 6 zeigen die beiden besten Bindungen bei einem Vergleich der Stellvertreter aller zehn Gruppen der Tabelle 1, festgestellt nach der in Beispiel 4 beschriebenen Methode. In der SEQ ID NO: 55 bedeutet X₁ entweder G oder L, und X₂ bedeutet entweder A oder L wobei L für kein Nukleotid (Deletion) steht. Positionen, an denen verschiedene Nukleotide stehen können, sind mit Unterstreichungen gekennzeichnet.

Die Tabelle 4 zeigt übereinstimmende Sequenzmotive beim Vergleich der Gruppen 1 und 8 der Tabelle 1. Die Stellvertreter der Gruppen 1 und 8 zeigen die besten (Gruppe 1) und drittbeste (Gruppe 8) Bindung bei einem Vergleich der Stellvertreter aller zehn Gruppen der Tabelle 1, festgestellt nach der in Beispiel 4 beschriebenen Methode. In der SEQ ID NO: 56 bedeutet X₂ entweder A oder L, und X₄ bedeutet G, A (=R) oder L, wobei L für kein Nukleotid (Deletion) steht. Positionen, an denen verschiedene Nukleotide stehen können, sind mit Unterstreichungen gekennzeichnet.

Die Tabelle 5 zeigt übereinstimmende Sequenzmotive beim Vergleich der Gruppen 6 und 8 der Tabelle 1. Die Stellvertreter der Gruppen 6 und 8 zeigen die zweitbeste (Gruppe 6) und drittbeste (Gruppe 8) Bindung bei einem Vergleich der Stellvertreter aller zehn Gruppen der Tabelle 1. Positionen, an denen verschiedene Nukleotide stehen können, sind mit Unterstreichungen gekennzeichnet.

Die Tabelle 6 zeigt übereinstimmende Sequenzmotive beim Vergleich der Gruppen 1, 6 und 8 der Tabelle 1. Die Stellvertreter der Gruppen 1, 6 und 8 zeigen die beste, zweitbeste und drittbeste Bindung bei einem Vergleich der Stellvertreter aller zehn Gruppen der Tabelle 1. Positionen, an denen verschiedene Nukleotide stehen können sind mit Unterstreichungen gekennzeichnet. In der SEQ ID NO: 58 bedeutet X₂ entweder A oder L, und X₄ bedeutet G, A (=R) oder L, wobei L für kein Nukleotid (Deletion) steht.

**Tabelle 3: Übereinstimmende Motive innerhalb der Aptamergruppen 1 und 6**

| **Sequenzmotiv (5' → 3')** | **SEQ ID NO** |
|---|---|
| ATACCAGCTTATTCAATT | 59 |
| AGATAGTAAGTGCAATCT | 60 |
| GKATX₁X₂GGCTCGATYRAGGTC | 55 |

**Tabelle 4: Übereinstimmende Motive innerhalb der Aptamergruppen 1 und 8**

| **Sequenzmotiv (5' → 3')** | **SEQ ID NO** |
|---|---|
| ATACCAGCTTATTCAATT | 59 |
| AGATAGTAAGTGCAATCT | 60 |
| GGATX₄X₂GGCTYGRTY | 56 |

**Tabelle 5: Übereinstimmende Motive innerhalb der Aptamergruppen 6 und 8**

| **Sequenzmotiv (5' → 3')** | **SEQ ID NO** |
|---|---|
| ATACCAGCTTATTCAATT | 59 |
| AGATAGTAAGTGCAATCT | 60 |
| GKATRAGGCTYGRTY | 57 |

**Tabelle 6: Übereinstimmende Motive innerhalb der Aptamergruppen 1, 6 und 8**

| **Sequenzmotiv (5' → 3')** | **SEQ ID NO** |
|---|---|
| ATACCAGCTTATTCAATT | 59 |
| AGATAGTAAGTGCAATCT | 60 |
| GKATX₄X₂GGCTYGRTY | 58 |

### BEISPIEL 4: Bestimmung der Spezifität einzelner Aptamerer.

Die erhaltenen Sequenzen der Aptamer-Klone konnten anhand ihrer Ähnlichkeit in zehn Gruppen eingeteilt werden. Aus jeder Gruppe wurde je ein Stellvertreter ausgewählt und bei Microsynth (Schweiz) chemisch hergestellt und PAGE gereinigt. Diese Stellvertreter wurden auf ihre individuelle Bindungsfähigkeit an jede der vier in der Targetmischung vorhandenen Einzelsubstanzen, sowie an die Mischung selbst, getestet. Als Negativkontrolle wurde parallel Puffer ohne enthaltene Targetsubstanz verwendet.

Für jeden Bindungstest wurde ein frisches Aliquot von 3 x 10⁸ der mit der Fängersequenz modifizierten Magnetic Beads (1 × 10⁷ Beads für jede Bindungsreaktion) zunächst 3 × in Bindungspufferlösung (BB(KC): 20 mmol L⁻¹ Tris-HCl pH 7.6, 100 mmol L⁻¹ NaCl, 2 mmol L⁻¹ MgCl₂, 5 mmol L⁻¹ KCl, 1 mmol L⁻¹ CaCl₂) gewaschen. Die Aptamer-ssDNA wurde vorbereitet, indem ca. 300 pmol in 500 µL BB(KC) für 8 min bei 90 °C inkubiert, danach 10 min auf Eis und 5-6 min bei Raumtemperatur äquilibriert wurden. Das vorbereitete Aptamer wurde mit dem gewaschenen Aliquot Fängersequenz-modifizierter Magnetic Beads über Nacht bei 21°C unter leichtem Schütteln inkubiert. Nach der Bindungsreaktion wurden die ungebundenen Aptamere entfernt und wie alle folgenden Fraktionen gesammelt.

Der Bindungskomplex wurde 9 × in jeweils 500 µL BB(KC) gewaschen. Die Magnetic Beads mit den gebundenen Aptameren wurden nun einem Temperaturschritt wie während des Selektionsprozesses unterworfen: Inkubation in 500 µL BB(KC) bei 28 °C für 15 min bei leichtem Schütteln. Aptamere, die sich während dieses Schrittes von den Magnetic Beads gelöst hatten, wurden entfernt. Anschließend wurden die Magnetic Beads 7 x mit je 500 µL BB(KC) gewaschen. Im Hintergrund-Elutionsschritt wurden die Magnetic Beads in 300 µL BB(KC) bei 21 °C für 45 min leicht geschüttelt. Anschließend wurden gelöste Aptamere entfernt. Die Beads wurden 6 x in je 500 µL BB(KC) gewaschen, dann in 600 µL BB(KC) aufgenommen und auf 6 vorgespülte Reaktionsgefäße gleichmäßig verteilt. Der Überstand wurde abgenommen und die einzelnen Aliquots in je 300 µL Targetsubstanz-Lösung, deren Mischung sowie Puffer ohne Targetsubstanz bei 21 °C 45 min inkubiert. Anschließend wurde die Lösung abgenommen. In der so entstandenen Fraktion der Target-Elution sollten sich diejenigen Aptamere befinden, welche sich durch Trennung der Fängersequenz-Aptamer-Bindung bei Anwesenheit der Targetsubstanz(en) von den Magnetic Beads gelöst hatten. Die danach noch an den Magnetic Beads gebundenen Aptamere wurden durch Hitze eluiert (2 x 300 µL BB(KC), 95 °C, 10 min). Die DNA-Mengen in allen so gewonnenen Fraktionen (inklusive der Waschschritte) wurden mittels Fluorometrie (Wallac Victor²V Multilabel Counter; PerkinElmer life sciences, Deutschland) bei folgenden Bedingungen gemessen: Excitation 485 nm/Emission 535 nm; time 1 s; CW-lamp energy 22500. Das Messvolumen war 100 µL/well in 96 well black Mikrotiterplatten (NUNC, Deutschland). Die Berechnung der ssDNA-Konzentration jeder Fraktion erfolgte unter Verwendung einer Kalibrierkurve, welche aus der jeweiligen Aptamer-DNA hergestellt wurde, die den gleichen thermischen Bedingungen bei Äquilibrierung und Über-Nacht-Inkubation ausgesetzt war wie die bei den Spezifitätstests eingesetzte Aptamer-DNA.

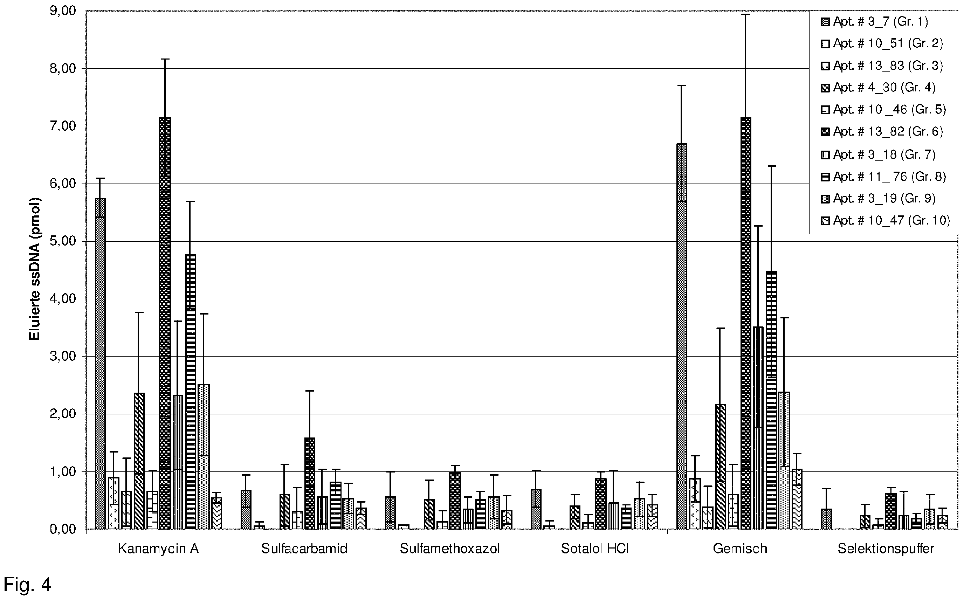

Die Fig. 4 zeigt einen Spezifitätstest für den Stellvertreter jeder der Gruppen der DNA-Oligomere, die aus dem Capture-SELEX gegen die Target-Mischung bestehend aus Kanamycin A Disulfatsalz Dihydrat, Sulfcarbamid, Sulfamethoxazol, und Sotalol-Hydrochlorid, jeweils 1 mM in Selektionspuffer, erhalten wurden. Die durchschnittliche Menge von 31,4 ± 11,2 pmol Oligonukleotide wurde auf (4,6 ± 0,6) * 10⁷ magnetischen Beads immobilisiert. In den Spezifitätstests wurde die Menge eluierter Oligomere in Gegenwart der Pharmazeutika bestimmt. Die Bedingungen wurden vergleichbar zu den Schritten der SELEX-Prozedur gewählt. Die vier Pharmazeutika wurden individuell in einer Konzentration von 1 mM in Selektionspuffer getestet, wie auch ihre Mischung und Selektionspuffer allein als Negativkontrolle. Die Menge eluierter ssDNA wurde auf 5 * 10⁷ Beads normalisiert. Die Experimente wurden im Fall von bindenden Sequenzen (Aptameren) zweifach oder dreifach durchgeführt. Die Ergebnisse zeigen, dass die Stellvertreter der Gruppen 1, 4, 6, 7, 8, 9 und 10 bindende Eigenschaften aufweisen.

### BEISPIEL 5: Aufbau eines Biosensors und Durchführung eines Nachweises auf Kanamycin A

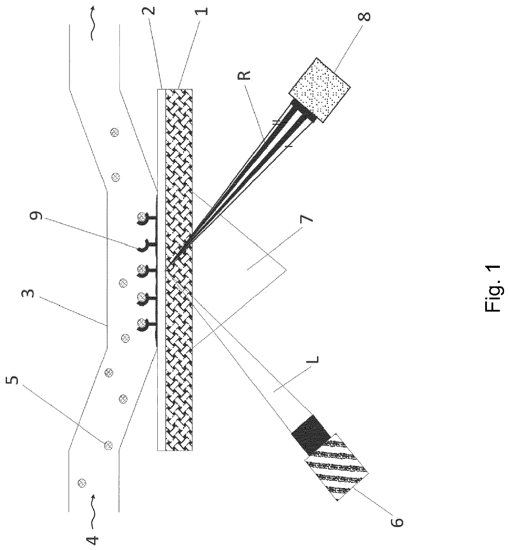

### Bezugszeichenliste zu Fig. 1:

- 1: Sensorchip (Glaschip)
- 2: Goldfilm
- 3: Flusszelle
- 4: Probelösung
- 5: Target
- 6: Lichtquelle
- 7: Prisma
- 8: Detektor
- 9: Aptamer
- L: monochromatisches polarisiertes Licht
- R: reflektiertes Licht

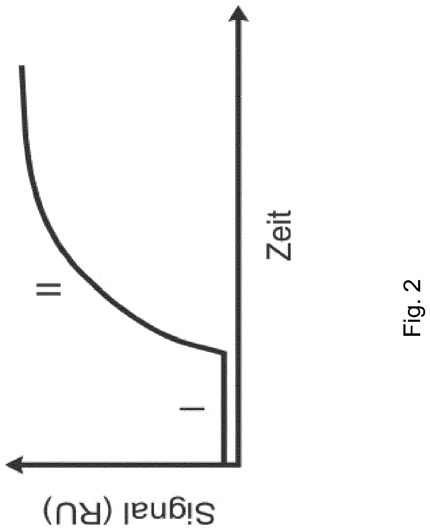

Das Biacore®-System ist ein kommerziell erhältliches Biosensor-System, welches den markierungsfreien Nachweis von biomolekularen Wechselwirkungen in Echtzeit ermöglicht und dafür das physikalische Prinzip der Oberflächenplasmonenresonanz-Spektroskopie (Surface Plasmon Resonance, SPR) nutzt, wie in der Fig. 1 dargestellt. Der Biosensorchip des Biacore-Systems besteht aus einem Glas-Chip 1, der mit einem dünnen Goldfilm 2 beschichtet ist und die Sensoroberfläche darstellt. Eine spezielle Funktionalisierung dieser Sensoroberfläche, beispielsweise mit Carboxymethyldextran (CM), erlaubt die kovalente Immobilisierung der biologischen Rezeptormoleküle. Im Falle der Aptamere 9 wird bevorzugt das Biotin-Streptavidin-Kopplungssystem genutzt, um die biotinylierten Aptamere auf die Streptavidin-beschichtete Sensoroberfläche des Biosensorchips zu immobilisieren. Die anschließende Interaktion zwischen Target und Aptamer findet in einer Flusszelle 3 statt, wobei durch ein integriertes, kontinuierliches Flusssystem die Lösung 4 mit Target 5 über die Sensoroberfläche geleitet wird. Die optische Detektionseinheit des Biacore-Systems besteht aus einer Lichtquelle 6 (Leuchtdioden), einem Prisma 7 und einem Detektor 8 (Diodenarray-Detektor). Monochromatisches, polarisiertes Licht L wird unter den Bedingungen der Totalreflexion von einem Medium mit hohem Brechungsindex (Sensorchip mit Goldfilm) in ein Medium mit niedrigem Brechungsindex (Sensorschicht, Pufferlösung) eingestrahlt. Dabei tritt bei einem bestimmten Einfallswinkel eine Abschwächung des reflektierten Lichtes auf. Dieser Winkel, der Resonanzwinkel, reagiert sehr sensitiv auf Veränderungen des Brechungsindexes der Sensorschicht. Die Anbindung des Targets 5 an das immobilisierte Aptamer 9 auf der Sensoroberfläche resultiert in einem Massezuwachs in der Sensorschicht, welcher zu einer Änderung des Brechungsindexes dieser Schicht und damit zu einer Verschiebung des Resonanzwinkels führt. Diese Änderungen werden als messbares Signal, ausgedrückt in Resonanz-Einheiten (RU), ausgegeben und sind proportional zu der Menge an gebundenem Target 5 auf der Sensoroberfläche. Während der Bindungsanalyse werden die Änderungen des Resonanzwinkels kontinuierlich aufgezeichnet und aufgetragen gegen die Zeit als Sensorgramm dargestellt, das in der Fig. 2 schematisch dargestellt ist. Im Abschnitt II der Signalkurve ist eine Verschiebung des Resonanzwinkels und die Anbindung von Aptamer erkennbar. Die Verschiebung des Resonanzwinkels ist auch in der Fig. 1 gezeigt, anhand eines mit "I" und eines mit "II" bezeichneten reflektierten Lichtstrahles.

Der Aptamer-Target-Komplex kann unter geeigneten Pufferbedingungen wieder gelöst werden, so dass die Sensoroberfläche regeneriert wird und für eine erneute Bindung mit Targetmolekülen zu Verfügung steht.

An der Oberfläche des oben beschriebenen Biosensors wird ein erfindungsgemäßes Aptamer immobilisiert. Eine Lösung von Kanamycin A wird über die Sensoroberfläche geleitet, wobei Kanamycin A an den Aptamer bindet. Die Anbindung von Kanamycin A wird als Verschiebung des Resonanzwinkels detektiert.

## Patentansprüche

1. Aptamer, das an eine oder mehrere Verbindungen aus der Gruppe der Aminoglykoside bindet, wobei das Aptamer ausgewählt ist aus der Gruppe bestehend aus
a) einem Aptamer umfassend eine Nukleinsäuresequenz, die ausgewählt ist aus der Gruppe bestehend aus SEQ ID NO: 45, 1-10, 50, 23-26, 52, 29-31, 48, 17-19, 51, 27, 28, 53, 32, 33, 54, 34, 35, und 55-58, mit der Maßgabe, dass Thymin durch Uracil ersetzt sein kann,
b) einem Aptamer, dessen Nukleinsäuresequenz eine Identität von mindestens 70% mit der Nukleinsäuresequenz eines Aptamers aus a) aufweist,
c) einem Aptamer, das mit dem komplementären Strang eines Aptamers aus a) unter stringenten Bedingungen hybridisiert,
d) einem Aptamer bei dem gegenüber einem Aptamer aus a) ein oder mehrere Nukleotide substituiert, deletiert, insertiert und/oder angefügt sind, wobei dieses Aptamer eine Identität von mindestens 70% mit der Nukleinsäuresequenz eines Aptamers aus a) aufweist,
e) einem Fragment eines Aptamers nach a), b), c) oder d), und
f) einem Derivat eines Aptamers nach a), b), c), d) oder e), das eine chemische Struktur aufweist, die in natürlicher DNA oder RNA nicht vorkommt.

2. Aptamersonde zur Detektion einer oder mehrerer Verbindungen aus der Gruppe der Aminoglykoside, umfassend ein oder mehrere verschiedene Aptamere wie in Anspruch 1 beschrieben und ein Markierungsmittel.

3. Biosensor, aufweisend ein oder mehrere verschiedene Aptamere wie in Anspruch 1 beschrieben.

4. Feste Phase, insbesondere zur Verwendung beim Nachweis, Anreicherung, Abtrennung und/oder Isolierung einer oder mehrerer Verbindungen aus der Gruppe der Aminoglykoside, an die ein oder mehrere verschiedene Aptamere nach Anspruch 1 immobilisiert ist/sind.

5. Teststreifen, insbesondere zur Verwendung in Lateralfluss-Assays, der ein oder mehrere verschiedene Aptamere nach Anspruch 1 enthält.

6. Lateralfluss-Assay-Vorrichtung, die einen Teststreifen nach Anspruch 5 aufweist.

7. Kit, umfassend ein oder mehrere verschiedene Aptamere wie in Anspruch 1 beschrieben.

8. Messgerät zum Nachweis von Verbindungen aus der Gruppe der Aminoglykoside, aufweisend ein oder mehrere verschiedene Aptamere nach Anspruch 1, eine Aptamersonde nach Anspruch 2, einen Biosensor nach Anspruch 3 oder einen Teststreifen nach Anspruch 5.

9. Verwendung des Aptamers wie in Anspruch 1 beschrieben zum Nachweis, Anreicherung, Abtrennung und/oder Isolierung einer oder mehrerer Verbindungen aus der Gruppe der Aminoglykoside.

10. Verfahren zum Nachweis einer oder mehrerer Verbindungen aus der Gruppe der Aminoglykoside, bei dem
a) ein oder mehrere verschiedene Aptamere wie in Anspruch 1 beschrieben mit einer Probe, die eine oder mehrere Verbindungen aus der Gruppe der Aminoglykoside enthält, in Kontakt gebracht wird/werden und
b) eine Bindung zwischen dem/den Aptamer(en) und der/den Verbindung(en) aus der Gruppe der Aminoglykoside nachgewiesen wird.

11. Verfahren nach Anspruch 10, das ein in Form eines kompetitiven Assays oder eines Sandwich-Assays durchgeführt wird.

12. Verfahren zur Anreicherung, Abtrennung und/oder Isolierung einer oder mehrerer Verbindungen aus der Gruppe der Aminoglykoside, bei dem
a) ein oder mehrere verschiedene Aptamere wie in Anspruch 1 beschrieben mit einer Probe, die eine oder mehrere Verbindungen aus der Gruppe der Aminoglykoside enthält, in Kontakt gebracht wird/werden, wobei ein Komplex oder Komplexe aus dem/den Aptamer(en) und der/den Verbindung(en) aus der Gruppe der Aminoglykoside gebildet wird,
b) der/die Komplex(e) und die übrige Probe voneinander getrennt werden, und
c) optional die Verbindung(en) aus der Gruppe der Aminoglykoside aus dem Komplex isoliert wird/werden.

13. Verfahren nach einem der Ansprüche 10-12, bei dem das/die Aptamer(e) an einer festen Phase immobilisiert ist/sind.

14. Verwendung einer Aptamersonde nach Anspruch 2, eines Biosensors nach Anspruch 3, einer festen Phase nach Anspruch 4, eines Teststreifens nach Anspruch 5, einer Lateralfluss-Assay-Vorrichtung nach Anspruch 6, eines Kits nach Anspruch 7, oder eines Messgeräts nach Anspruch 8 zum Nachweis einer oder mehrerer Verbindungen aus der Gruppe der Aminoglykoside oder zur Anreicherung, Abtrennung und/oder Isolierung einer oder mehrerer Verbindungen aus der Gruppe der Aminoglykoside.

## Claims

1. An aptamer that binds to one or more compounds from the group of the aminoglycosides, the aptamer being selected from the group consisting of
a) an aptamer comprising a nucleic acid sequence that is selected from the group consisting of SEQ ID NO: 45, 1-10, 50, 23-26, 52, 29-31, 48, 17-19, 51, 27, 28, 53, 32, 33, 54, 34, 35, and 55-58, with the proviso that thymine can be replaced by uracil,
b) an aptamer having a nucleic acid sequence identity of at least 70% with the nucleic acid sequence of an aptamer from a),
c) an aptamer that hybridizes under stringent conditions with the complementary strand of an aptamer from a),
d) an aptamer in which one or more nucleotides have been substituted, deleted, inserted, and/or added in comparison with an aptamer from a), this aptamer having a nucleic acid sequence identity of at least 70% with the aptamer from a),
e) a fragment of an aptamer according to a), b), c), or d), and
f) a derivative of an aptamer according to a), b), c), d), or e) that has a chemical structure that does not occur in natural DNA or RNA.

2. An aptamer probe for detection of one or more compounds from the group of the aminoglycosides, comprising one or more different aptamers described in claim 1 and a means of labeling.

3. A biosensor having one or more different aptamers as described in claim 1.

4. A solid phase especially for use in the detection, enrichment, separation and/or isolation of one or more compounds from the group of the aminoglycosides, this solid phase having one or more different aptamers according to claim 1 immobilized on it.

5. A test strip especially for use in lateral flow assays, this test strip containing one or more different aptamers according to claim 1.

6. A lateral flow assay device that has a test strip according to claim 5.

7. A kit comprising one or more different aptamers described in claim 1.

8. A measuring instrument to detect compounds from the group of aminoglycosides, having one or more different aptamers according to claim 1, an aptamer probe according to claim 2, a biosensor according to claim 3, or a test strip according to claim 5.

9. The use of an aptamer described in claim 1 for the detection, enrichment, separation and/or isolation of one or more compounds from the group of the aminoglycosides.

10. A process for the detection of one or more compounds from the group of the aminoglycosides, involving
a) putting one or more different aptamers described in claim 1 in contact with a probe containing one or more compounds from the group of aminoglycosides; and
b) detecting a binding between the aptamer(s) and the compound(s) from the group of aminoglycosides.

11. A process according to claim 10 that is carried out in the form of a competitive assay or a sandwich assay.

12. A process for the enrichment, separation, and/or isolation of one or more compounds from the group of the aminoglycosides, involving
a) putting one or more different aptamers described in claim 1 in contact with a probe containing one or more compounds from the group of aminoglycosides, forming a complex or complexes of the aptamer(s) and the compound(s) from the group of aminoglycosides,
b) separating the complex(es) and the rest of the probe from one another; and
c) optionally isolating the compound(s) from the group of the aminoglycosides from the complex.

13. A process according to one of claims 10-12, in which the aptamer(s) is/are immobilized on a solid phase.

14. The use of an aptamer probe according to claim 2, a biosensor according to claim 3, a solid phase according to claim 4, a test strip according to claim 5, a lateral flow assay device according to claim 6, a kit according to claim 7, or a measuring instrument according to claim 8 for the detection of one or more compounds from the group of the aminoglycosides or for the enrichment, separation and/or isolation of one or more compounds from the group of the aminoglycosides.

## Revendications

1. Aptamère, qui se lie à un ou à plusieurs composés issus du groupe des aminoglycosides, dans lequel l'aptamère est choisi parmi le groupe constitué
a) d'un aptamère comprenant une séquence d'acide nucléique, qui est choisie parmi le groupe constitué de SEQ ID NO : 45, 1-10, 50, 23-26, 52, 29-31, 48, 17-19, 51, 27, 28, 53, 32, 33, 54, 34, 35 et 55-58, à condition que la thymine puisse être remplacée par l'uracile,
b) d'un aptamère, dont la séquence d'acide nucléique présente une identité d'au moins 70 % avec la séquence d'acide nucléique d'un aptamère issu de a),
c) d'un aptamère, qui est hybridé par le brin complémentaire d'un aptamère issu de a) dans des conditions stringentes,
d) d'un aptamère, où un ou plusieurs nucléotides sont substitués, supprimés, insérés et/ou ajoutés par rapport à un aptamère issu de a), dans lequel ledit aptamère présente une identité d'au moins 70 % avec la séquence d'acide nucléique d'un aptamère issu de a),
e) d'un fragment d'un aptamère selon a), b), c) ou d), et
f) d' un dérivé d' un aptamère selon a), b), c), d) ou e), qui présente une structure chimique, qui n'est pas présente dans l'ADN ou l'ARN naturel.

2. Sonde d'aptamère servant à détecter un ou plusieurs composés issus du groupe des aminoglycosides, comprenant un ou plusieurs aptamères différents tels que décrits dans la revendication 1 et un moyen de marquage.

3. Biocapteur, présentant un ou plusieurs aptamères différents tels que décrits dans la revendication 1.

4. Phase solide, en particulier destinée à être utilisée lors de la démonstration de la présence d'un ou de plusieurs composés, de l'enrichissement, de la séparation et/ou de l'isolation d'un ou de plusieurs composés issus du groupe des aminoglycosides, au niveau duquel un ou plusieurs aptamères différents selon la revendication 1 est immobilisé/sont immobilisés.

5. Bandelette de test, en particulier destinée à être utilisée dans un essai de flux latéral, qui contient un ou plusieurs aptamères différents selon la revendication 1.

6. Dispositif d'essai de flux latéral, qui présente une bandelette de test selon la revendication 5.

7. Kit, comprenant un ou plusieurs aptamères différents tels que décrits dans la revendication 1.

8. Appareil de mesure servant à démontrer la présence de composés issus du groupe des aminoglycosides, présentant un ou plusieurs aptamères différents selon la revendication 1, une sonde d'aptamère selon la revendication 2, un biocapteur selon la revendication 3 ou une bandelette de test selon la revendication 5.

9. Utilisation de l'aptamère tel que décrit dans la revendication 1 servant à enrichir, séparer et/ou isoler un ou de plusieurs composés issus du groupe des aminoglycosides, à démontrer leur présence.

10. Procédé servant à démontrer la présence d'un ou de plusieurs composés issus du groupe des aminoglycosides, où
a) un ou plusieurs aptamères différents tels que décrits dans la revendication 1 est amené/sont amenés en contact avec un échantillon, qui contient un ou plusieurs composés issus du groupe des aminoglycosides, et
b) une liaison entre le/les aptamère(s) et le/les composé(s) issus du groupe des aminoglycosides est démontrée.

11. Procédé selon la revendication 10, qui est mis en oeuvre sous la forme d'un essai compétitif ou d'un essai en sandwich.

12. Procédé servant à enrichir, séparer et/ou isoler un ou plusieurs composés issus du groupe des aminoglycosides, où
a) un ou plusieurs aptamères différents tels que décrits dans la revendication 1 est amené/sont amenés en contact avec un échantillon, qui contient un ou plusieurs composés issus du groupe des aminoglycosides, dans lequel un complexe ou des complexes issu(s) du/des aptamère(s) et du/des composé(s) est/sont formé(s) à partir du groupe des aminoglycosides,
b) le/les complexe(s) et l'échantillon restant sont séparés les uns des autres, et
c) en option le/les composé(s) issu(s) du groupe des aminoglycodises est/sont isolé(s) du complexe.

13. Procédé selon l'une quelconque des revendications 10 - 12, où le/les aptamère(s) est/sont immobilisé(s) au niveau d'une phase solide.

14. Utilisation d'une sonde d'aptamère selon la revendication 2, d'un biocapteur selon la revendication 3, d'une phase solide selon la revendication 4, d'une bandelette de test selon la revendication 5, d'un essai de flux latéral selon la revendication 6 d'un kit selon la revendication 7, ou d'un appareil de mesure selon la revendication 8 afin de démontrer la présence d'un ou de plusieurs composés issus du groupe des aminoglycosides ou afin d'enrichir, de séparer et/ou d'isoler un ou plusieurs composés issus du groupe des aminoglycosides.
